# EUROPEAN PATENT SPECIFICATION

(11) **EP 2 509 967 B1**
(45) Date of publication and mention of the grant of the patent: **17.02.2016**
(21) Application number: 10790400.5
(22) Date of filing: 07.12.2010
(51) Int. Cl.: C07D 273/00, A61K 31/395, A61P 33/00

(54) **NOVEL 24-MEMBERED CYCLOOCTADEPSIPEPTIDES FROM FUNGAL STRAINS AND THEIR USE AS ANTHELMINTICS OR ENDOPARASITICIDES**
NEUE 24-GLIEDRIGE CYCLOOCTADEPSIPEPTIDE AUS PILZSTÄMMEN UND IHRE VERWENDUNG ALS ANTHELMINTIKA ODER ENDOPARASITIZIDE
NOUVEAUX CYCLOOCTADEPSIPEPTIDES À 24 CHAÎNONS ISSUS DE SOUCHES FONGIQUES ET LEUR UTILISATION COMME ANTHELMINTHIQUES OU ENDOPARASITICIDES

(30) Priority: 11.12.2009 EP 09178799
(43) Date of publication of application: 17.10.2012
(73) Proprietor: Bayer Intellectual Property GmbH, 40789 Monheim (DE)
(72) Inventor: HARDER, Achim, 51109 Köln (DE); KRIEGER, Klemens, 51789 Lindlar (DE); PHAM, Thi, Lam, Huong, 10117 Berlin (DE); HUYNH, Thanh, Dam, 10587 Berlin (DE); ZASPEL, Irmtraut, 15374 Müncheberg (DE); EWALD, Dietrich, 15518 Gölsdorf (DE); MÜGGE, Clemens, 10099 Berlin (DE); WEIßHOFF, Hardy, 13088 Berlin (DE)
(74) Representative: BIP Patents
(86) International application number: PCT/EP2010/069040
(87) International publication number: WO 2011/069995

(56) References cited:
- EP-A1- 0 930 304
- EP-A2- 0 382 173
- WO-A1-2010/072323
- SUZUKI A ET AL: "Bassianolide, a new insecticidal cyclodepsipeptide from Beauveria bassiana and Verticillium lecanii", TETRAHEDRON LETTERS, ELSEVIER, AMSTERDAM, NL, vol. 18, no. 25, 1 January 1977 (1977-01-01), pages 2167-2170, XP027190719, ISSN: 0040-4039 [retrieved on 1977-01-01]

## Description

The present invention relates firstly to the discovery of the novel 24-membered cyclooctadepsipeptides (the previously unknown derivatives PF1022-V and PF1022-W of the known anthelmintic cyclooctadepsipeptide PF1022-A and the previously unknown derivatives XRB-C894, XRB-C942, XRB-C976, XRB-C1010, XRB-C1044, XRB-E922, XRB-E956, XRB-E990, XRB-E1024, XRB-S958, XRB-S992, XRB-S1026, XRB-S1060 of the known cyclooctadepsipeptide bassianolide) and the processes for the preparation of the abovementioned cyclooctadepsipeptides by means of the fungal strains from the family *Xylariaceae,* in particular the genera *Rosellinia* and *Coniolariella,* and by means of the mitosporic fungal strains from the groups *Fusarium, Beauveria* and *Verticillium* (orders *Hypocreales* and *Phyllachorales),* and by means of the enzymatic preparations isolated from these fungal strains, and secondly the use of bassianolide and of the abovementioned cyclooctadepsipeptides, individually or as a mixture, as anthelmintics or endoparasiticides.

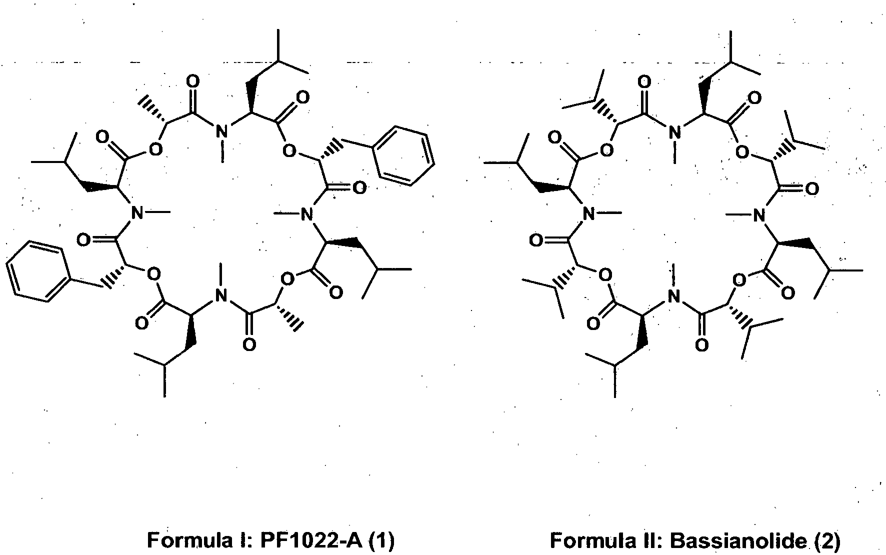

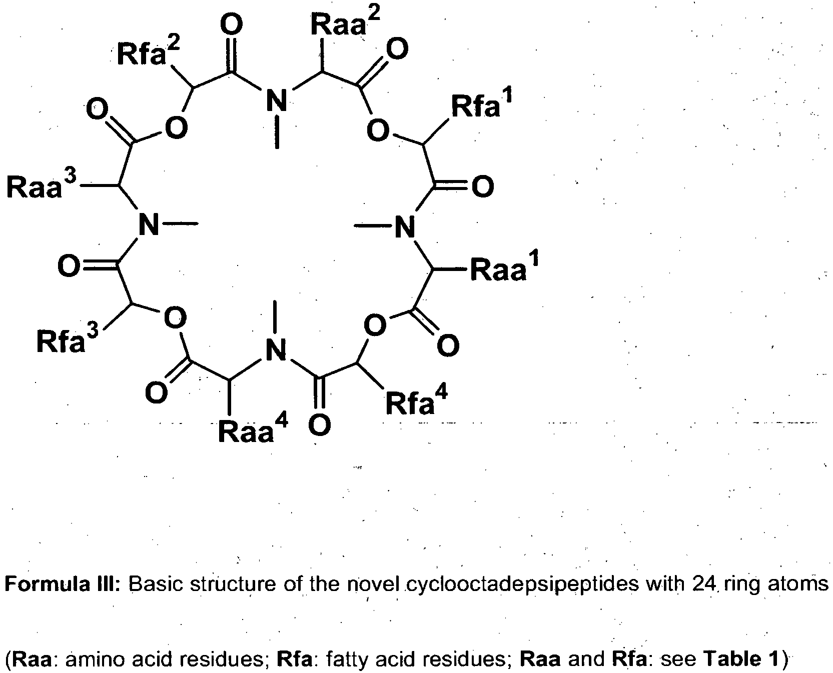

**Table 1: Amino acid residues (Raa) and fatty acid residues (Rfa) of the novel 24-membered cyclooctadepsipeptides (Nos. 3 to 17)**

| **Comp. No.** | **Name** | **Raa¹** | **Rfa¹** | **Raa²** | **Rfa²** | **Raa³** | **Rfa³** | **Raa⁴** | **Rfa⁴** |
|---|---|---|---|---|---|---|---|---|---|
| 1 | **PF1022-A** | -CH₂CH(CH₃)₂ | -CH₃ | -CH₂CH(CH₃)₂ | -CH₂C₆H₅ | -CH₂CH(CH₃)₂ | -CH₃ | -CH₂CH(CH₃)₂ | -CH₂C₆H₅ |
| 2 | **Bassianolide** | -CH₂CH(CH₃)₂ | -CH(CH₃)₂ | -CH₂CH(CH₃)₂ | -CH(CH₃)₂ | -CH₂CH(CH₃)₂ | -CH(CH₃)₂ | -CH₂CH(CH₃)₂ | -CH(CH₃)₂ |
| 3 | **PF1022-V⁽¹⁾** | -CH(CH₃)₂ | -CH₃ | -CH₂CH(CH₃)₂ | -CH₂C₆H₅ | -CH₂CH(CH₃)₂ | -CH₃ | -CH₂CH(CH₃)₂ | -CH₂C₆H₅ |
| 4 | **PF1022-W** | -CH₂CH(CH₃)₂ | -H | -CH₂CH(CH₃)₂ | -CH₂C₆H₅ | -CH₂CH(CH₃)₂ | -CH₃ | -CH₂CH(CH₃)₂ | -CH₂C₆H₅ |
| 5 | **XRB-C894** | -CH(CH₃)₂ | -CH(CH₃)₂ | -CH₂CH(CH₃)₂ | -CH(CH₃)₂ | -CH₂CH(CH₃)₂ | -CH(CH₃)₂ | -CH₂CH(CH₃)₂ | -CH(CH₃)₂ |
| 6 | **XRB-C942** | -CH₂C₆H₅ | -CH(CH₃)₂ | -CH₂CH(CH₃)₂ | -CH(CH₃)₂ | -CH₂CH(CH₃)₂ | -CH(CH₃)₂ | -CH₂CH(CH₃)₂ | -CH(CH₃)₂ |
| 7 | **XRB-C976⁽¹⁾** | -CH₂C₆H₅ | -CH(CH₃)₂ | -CH₂CH(CH₃)₂ | -CH(CH₃)₂ | -CH₂C₆H₅ | -CH(CH₃)₂ | -CH₂CH(CH₃)₂ | -CH(CH₃)₂ |
| 8 | **XRB-C1010⁽¹⁾** | -CH₂C₆H₅ | -CH(CH₃)₂ | -CH₂C₆H₅ | -CH(CH₃)₂ | -CH₂C₆H₅ | -CH(CH₃)₂ | -CH₂CH(CH₃)₂ | -CH(CH₃)₂ |
| 9 | **XRB-C1044⁽²⁾** | -CH₂C₆H₅ | -CH(CH₃)₂ | -CH₂C₆H₅ | -CH(CH₃)₂ | -CH₂C₆H₅ | -CH(CH₃)₂ | -CH₂C₆H₅ | -CH(CH₃)₂ |
| 10 | **XRB-E922** | -CH₂CH(CH₃)₂ | -C₄H₉ | -CH₂CH(CH₃)₂ | -CH(CH₃)₂ | -CH₂CH(CH₃)₂ | -CH(CH₃)₂ | -CH₂CH(CH₃)₂ | -CH(CH₃)₂ |
| 11 | **XRB-E956⁽¹⁾** | -CH₂C₆H₅ | -C₄H₉ | -CH₂CH(CH₃)₂ | -CH(CH₃)₂ | -CH₂CH(CH₃)₂ | -CH(CH₃)₂ | -CH₂CH(CH₃)₂ | -CH(CH₃)₂ |
| 12 | **XRB-E990⁽¹⁾** | -CH₂C₆H₅ | -C₄H₉ | -CH₂CH(CH₃)₂ | -CH(CH₃)₂ | -CH₂C₆H₅ | -CH(CH₃)₂ | -CH₂CH(CH₃)₂ | -CH(CH₃)₂ |
| 13 | **XRB-E1024^{(1,2)}** | -CH₂C₆H₅ | -C₄H₉ | -CH₂C₆H₅ | -CH(CH₃)₂ | -CH₂C₆H₅ | -CH(CH₃)₂ | -CH₂CH(CH₃)₂ | -CH(CH₃)₂ |

| **Comp. No.** | **Name** | **Raa¹** | **Rfa¹** | **Raa²** | **Rfa²** | **Raa³** | **Rfa³** | **Raa⁴** | **Rfa⁴** |
|---|---|---|---|---|---|---|---|---|---|
| 14 | **XRB-S958** | -(CH₂)₂SO₂CH₃ | -CH(CH₃)₂ | -CH₂CH(CH₃)₂ | -CH(CH₃)₂ | -CH₂CH(CH₃)₂ | -CH(CH₃)₂ | -CH₂CH(CH₃)₂ | -CH(CH₃)₂ |
| 15 | **XRB-S992⁽¹⁾** | -(CH₂)₂SO₂CH₃ | -CH(CH₃)₂ | -CH₂CH(CH₃)₂ | -CH(CH₃)₂ | -CH₂C₆H₅ | -CH(CH₃)₂ | -CH₂CH(CH₃)₂ | -CH(CH₃)₂ |
| 16 | **XRB-S1026^{(1,2)}** | -(CH₂)₂SO₂CH₃ | -CH(CH₃)₂ | -CH₂C₆H₅ | -CH(CH₃)₂ | -CH₂C₆H₅ | -CH(CH₃)₂ | -CH₂CH(CH₃)₂ | -CH(CH₃)₂ |
| 17 | **XRB-S1060⁽²⁾** | (CH₂)₂SO₂CH₃ | -CH(CH₃)₂ | -CH₂C₆H₅ | -CH(CH₃)₂ | -CH₂C₆H₅ | -CH(CH₃)₂ | -CH₂C₆H₅ | -CH(CH₃)₂ |

| | | | | | | | | | |
|---|---|---|---|---|---|---|---|---|---|
| ⁽¹⁾ including various structural isomers ⁽²⁾ occurs at very low concentrations | | | | | | | | | |

PF1022A (see formula I) is outstandingly suitable for controlling endoparasites, especially in the field of human and veterinary medicine. Such cyclooctadepsipeptides with 24 ring atoms and their use as endoparasiticides are already the subject matter of an earlier patent application (US005571793A).

Bassianolide (see formula II) has become known as an antibiotic with insecticidal activity (Kanaoka, M. et al., Bassianolide, a New Insecticidal Cyclodepsipeptide from Beauveria bassiana and Verticillium lecanii; Agric. Biol. Chem. 42 (1978), 629-635). A series of chemical and microbial processes exists for the preparation of cyclic, D-2-hydroxyisovaleric acid-containing depsipeptides with 24 ring atoms (for example by means of synthesis, cf.: Ohyama M. et al., Blosci. Blotechnol. Biochem. 58 (1994) p. 1193-1194; Scherkenbeck J. et al Tetrahedron 51 (1995) p. 8459-8470; Kobayashi M. et al. Annu, Rep. Sankyo Res. Lab. 46 (1994) p. 67-75; Lee B. et a/. Bioorg. Med. Chem. Lett. 12 (2002) p. 353-356; Dutton FE et al. J. Antibiot. 47 (1994) p. 1322-1327).

EP0382173 discloses the PF 1022 substance, a method of producing the same and anthelmintic composition containing the same. EP0930304 relates to a process for producing cyclodepsipeptide compounds and novel cyclodepsipeptides.

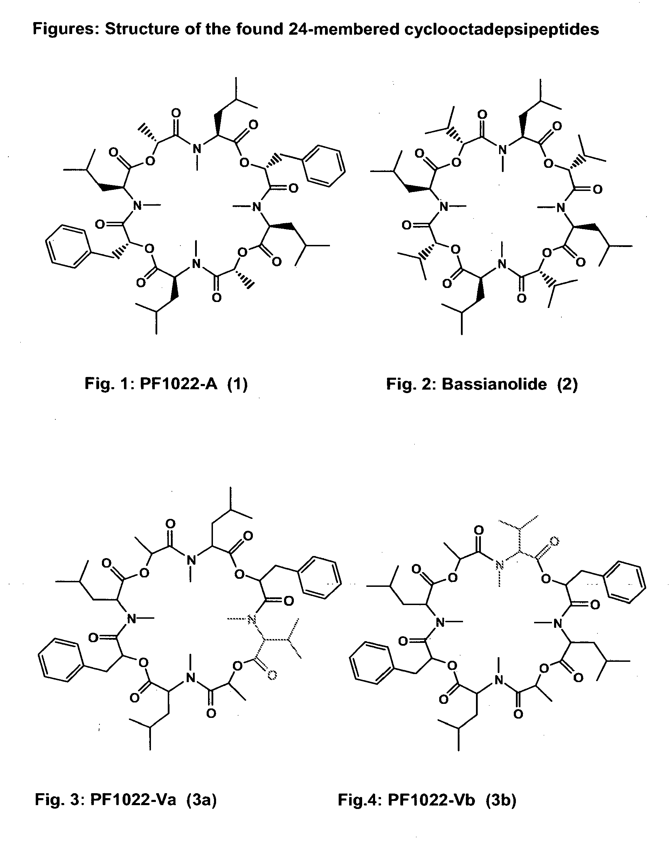

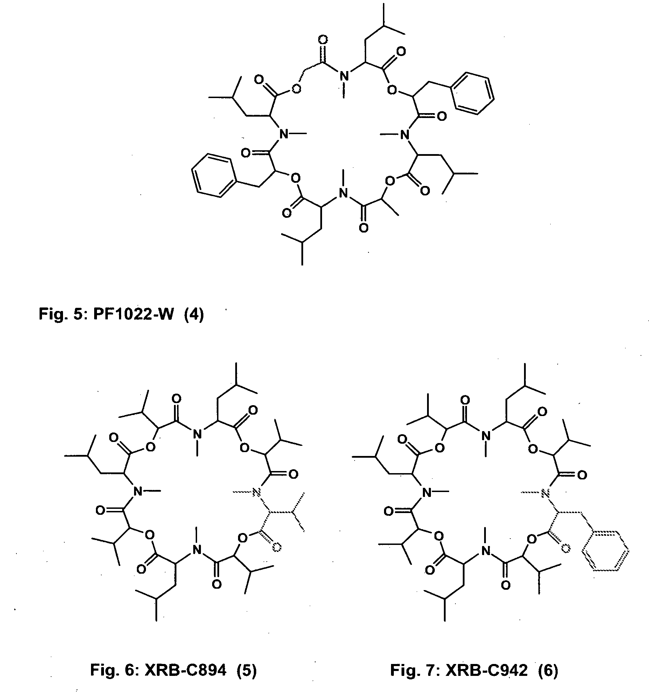

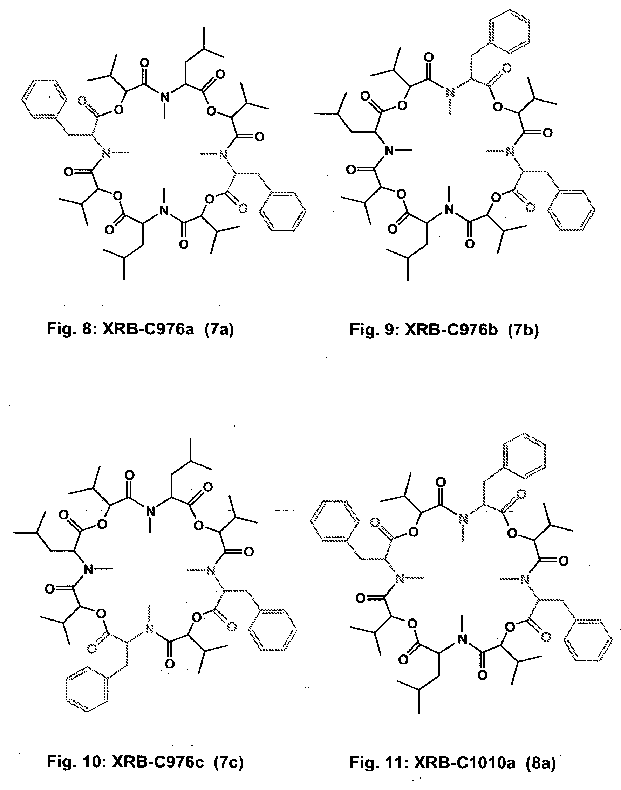

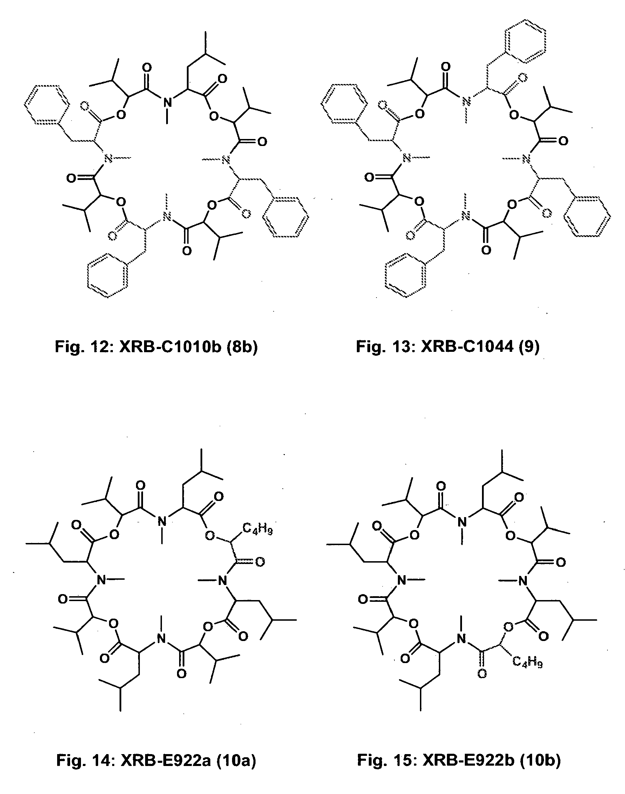

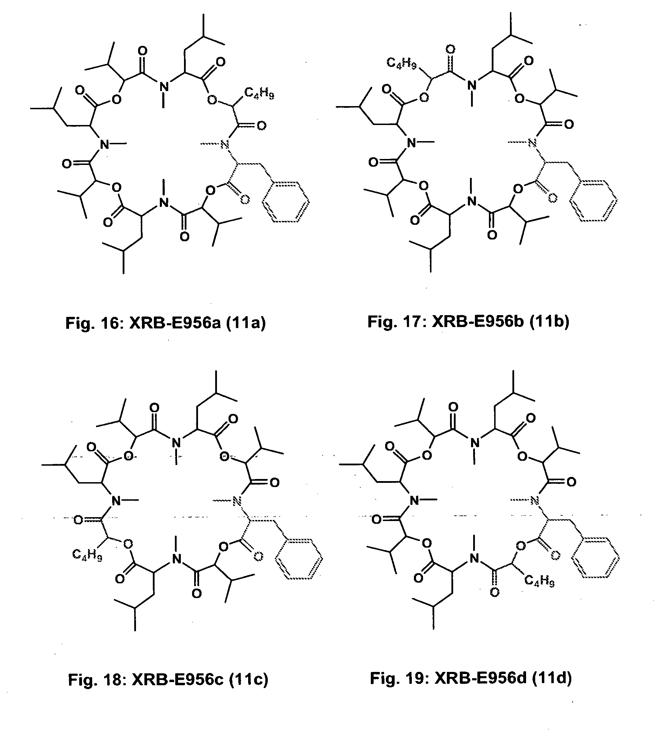

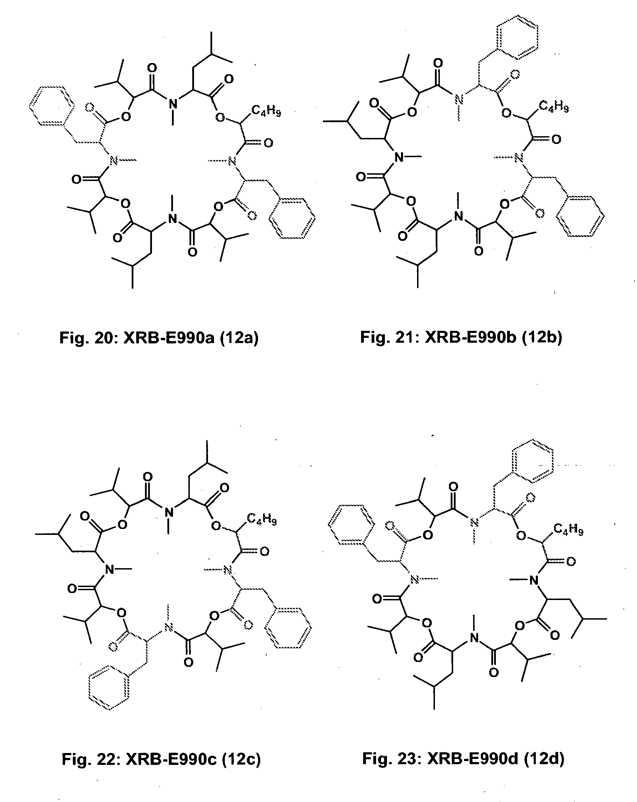

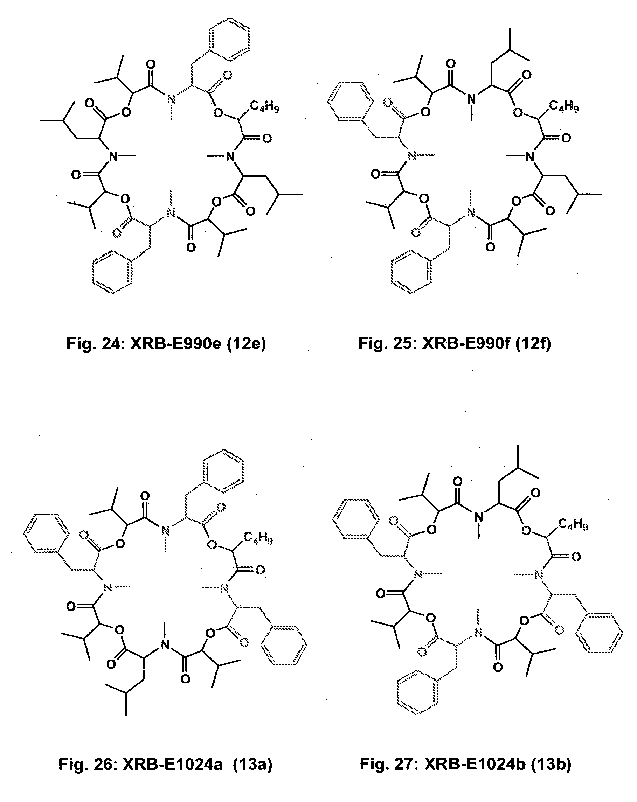

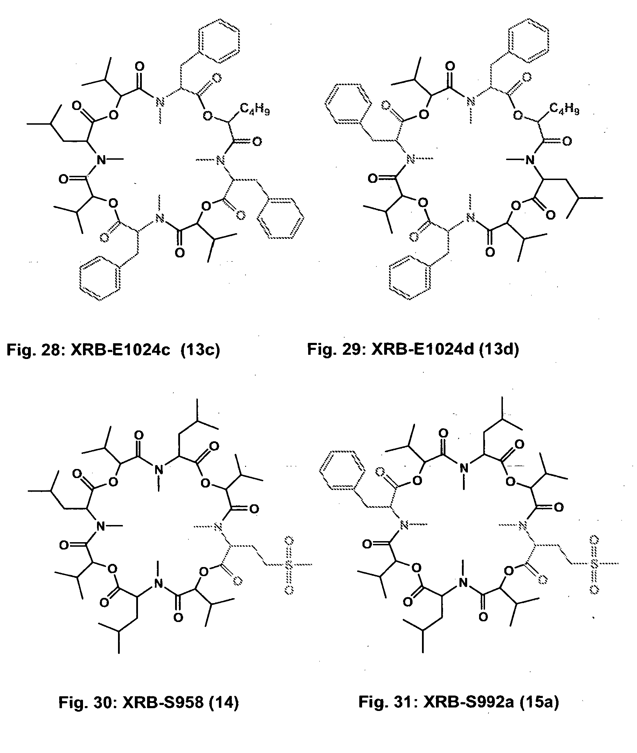

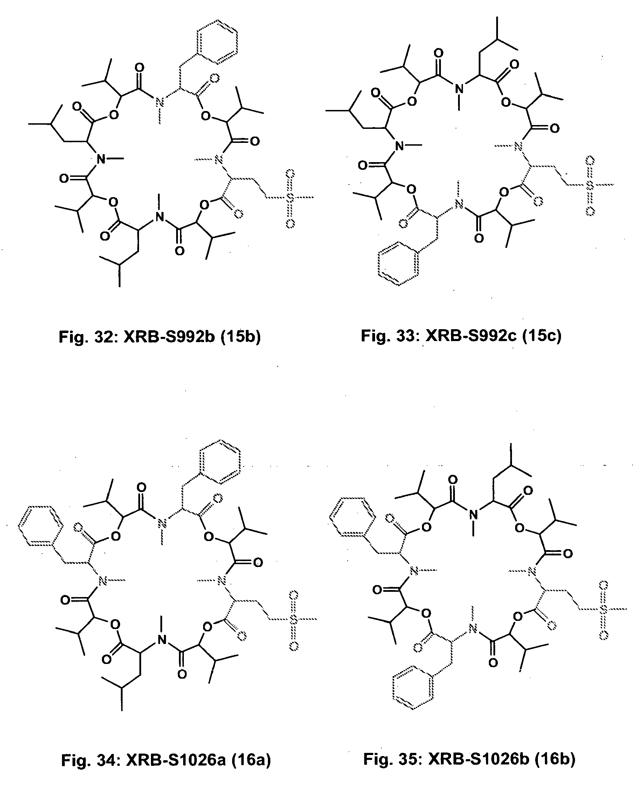

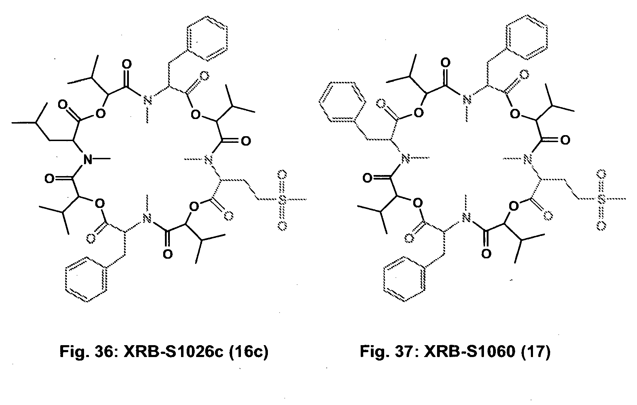

The present invention relates to the discovery of the previously unknown 24-membered cyclooctadepsipeptides (PF1022-V, PF1022-W, XRB-C894, XRB-C942, XRB-C976, XRB-C1010, XRB-C1044, XRB-E922, XRB-E956, XRB-E990, XRB-E1024, XRB-S958, XRB-S992, XRB-S1026, XRB-S1060) in various isomeric forms (see formula III, Table 1 and Figs. 3 to 37) and to the processes for the preparation of the abovementioned cyclooctadepsipeptides from the fungal strains from the family *Xylariaceae,* in particular the genera *Rosellinia* and *Coniolariella;* which are obtained from fruiting bodies of these representatives and further Xylariaceae in dead timber of broad-leaved trees and coniferous trees or from purchased, known strains of strain collections by multiple selections, and from the mitosporic fungal strains from the groups *Fusarium, Beauveria* and *Verticillium,* (orders *Hypocreales* and *Phyllachorales*), and by means of the enzymatic preparations isolated from these abovementioned fungal strains.

### Description of the generation or isolation of fungal strains or fungal lines as effective producers of novel 24-membered cyclooctadepsipeptides:

The present invention discloses the preparation of novel 24-membered cyclooctadepsipeptides (PF1022-V, PF1022-W, XRB-C894, XRB-C942, XRB-C976, XRB-C1010, XRB-C1044, XRB-E922, XRB-E956, XRB-E990, XRB-E1024, XRB-S958, XRB-S992, XRB-S1026, XRB-S1060) in various isomeric forms by means of the fungal strains from the family *Xylariaceae,* in particular the genera *Rosellinia* and *Coniolariella,* within which several fungal lines are capable of producing the cyclooctadepsipeptides.

Fungal strains from the family *Xylariaceae,* in particular the genus *Rosellinia,* were purchased from various strain collections in order to find novel cyclooctadepsipeptide producers. In order to obtain suitable fungal lines as efficient cyclooctadepsipeptide producers, the abovementioned fungal strains were improved in respect of the cyclooctadepsipeptide production by means of multiple selections. Furthermore, new isolates were obtained from fruiting bodies of *Rosellinia* and *Coniolariella* and from other Xylariaceae in the dead timber of various broad-leaved and coniferous tree species, and novel lines were obtained from these new isolates after selections in respect of their cyclooctadepsipeptide production. A variety of mitosporic fungi from the orders *Hypocreales* and *Fhyllachorales,* in particular the groups *Fusarium, Beauveria* and *Verticillium,* may occur as accompanying organisms, and these are likewise capable of producing cyclooctadepsipeptides.

The *fungal strains Rosellinia abscondita* (*CBS* 447.89, CBS 448.89, CBS 450.89), *R. britannica* (CBS 446.89), *R. mammaeformis* (CBS 445.89), *R. millegrana* (CBS 111.75), *R. nectrioides* (CBS 449.89), *R. necatrix* (CBS 267.30), *Xylaria hypoxylon* (CBS 499.80) were deposited in 1930, 1975 or 1989 (according to the CBS code) at the strain collection of the CBS (Centraalbureau voor Schimmelcultures, Institute of the Royal Netherlands Academy of Arts and Sciences, the Netherlands) and obtained therefrom in the form of cultures. The lines XR-909, XR-912, XR-913, XR-916 and XR-917, which differ in culture from the original strains by their growth habit and their capability for producing cyclooctadepsipeptides, were obtained from these CBS fungal strains after repeated selections on mSeed medium. The property of mycelial development is linked to the production of certain cyclooctadepsipeptides. Other fungal strains were isolated from natural sources.

The genus *Rosellinia* is a ubiquitous fungal genus of the family *Xylariaceae,* which belongs to the extensive order *Xylariales* (phylum *Ascomycota*).

The representatives of the genus *Rosellinia* are found on the bark of broad-leaved and coniferous trees in coolish locations of the moderate zones of the northern hemisphere. They live predominantly saprophytically on old or dead timber which is already in the process of degradation, or on herbaceous plants. They have characteristic spherical hard fruiting bodies, so-called perithecia with ostioles, in which asci with the species-typical spores develop. The asci are characterized by species-typical cubical or rectangular apical apparatuses which serve for ejecting the mature spores. The spores of the genus *Rosellinia* have species-typical germination slits.

The spores of *R. abscondita* are pale brown and between 16 to 24 x 5.5 to 8.5 µm in size. Very frequently, the germination slit is arranged diagonally over the spore surface. The spore poles bear a mucous sheath. The spores of *R. britannica* are 22 to 27 x 7 to 9 µm in size, brown and bear mucous sheaths. The spores of *R*. *mammaeformis* are 17 to 21 x 6 to 7 µm in size, brown with darker ends. The species *R. nectrioides* is very similar to *R*. *abscondita.* The asco spores are likewise pale brown, but there are no appendages or mucous sheaths.

The perithecia of the species are usually embedded in a dark brown to black subiculum (thick layers of hyphae). The hyphae grow from the subiculum into the bark tissue and the layers therebelow. The individual species make high demands on the environmental conditions which occur under natural conditions. Mature perithecia occur in mild winters and in the early months of the year. In general, the abovementioned *Rosellinia* species are rare.

The genus *Coniolariella* was renamed after various species, which had previously been classified under *Rosellinia,* had been classified as belonging to this genus (Checa et al. (2008) Mycol. Res. 112, 7, 795-801).

Obtaining pure cultures from natural habitats: Pure cultures (from fruiting bodies of *Rosellinia, Coniiolariella* and further Xylariaceaen in dead timber of broad-leaved and coniferous trees) were obtained from mature, intact perithecia which were as isolated as possible and which had been removed from dead timber. They were surface-disinfected with 0.05% strength AgNO₃ solution and rinsed repeatedly with sterile water. The perithecia were carefully crushed on a glass slide, and the asci and spores which were released were transferred into tubes containing sterile water.

By viewing under the microscope, it was possible to estimate the density and maturity stage at which the spores occurred. If the spore density was high, the spore suspension was diluted by a factor of ten, and 100 µl of the respective dilution stage were plated onto malt extract agar (MEA). The plates were stored at 18 to 20°C, scored every day under the microscope, and germinating spores were transferred to fresh potato dextrose agar (PDA). The mycelium formed which grows on this medium is whitish, flat and delicate; no aerial mycelium develops.

Besides the representatives of Xylariaceae, various mitosporic fungi which were classified as belonging, inter alia, to the groups *Fusarium, Verticillium* and *Beauveria,* grow at different densities. Pure cultures of these forms were likewise established.

After the cultures had grown sufficiently (colony sizes of about 85 mm), the mycelium was extracted with methanol. The constituents of the mycelial extracts were identified by means of LC-PDA-ESI-Q-TOF-MS and -MS/MS. Various lines of *Rosellinia* and *Coniolariella,* which proved to be positive in respect of the production of 24-membered cyclooctadepsipeptides, in particular of PF1022-A (see patent application BHC 08 8 004 = WO2010/072323, 16.12.2008) and of its derivatives PF1022-V and PF1022-W, were found. These include, for example, strain XR-21 (*Rosellinia corticum* from broad-leaved wood).

Since the present cultures are isolates which have been transferred directly from the natural ecosystem to artificial conditions, it is possible that culture forms appear which develop different mycelial forms and a variation in the intensity of the production of active substance. This is why the isolates obtained were improved by multiple selections in order to find cyclooctadepsipeptide production strains.

The fungal lines were selected for further propagation on the basis of the cyclooctadepsipeptide content, the cell growth and the mycelial form. Multiple selections and passages gave rise to fungal lines with a typical mycelial form, more rapid growth and better cyclooctadepsipeptide production capacity.

Assaying the growth of fungal lines: The growth of the fungal lines of the Xylariaceae in culture on malt extract agar (MEA), potato dextrose agar (PDA), cornmeal agar (CMA) or mSeed agar tends to be slow and to a high degree temperature-dependent. They develop a whitish flat delicate mycelium, in some cases also a filamentous mycelium, on these media.

In contrast to the original cultures, only a small amount of aerial mycelium is developed. It is only in older cultures that a white fine felty surface is formed, while a dark leather-like layer develops in agar. Line XR-909 is an exception in as far as the colour of the mycelium changes relatively rapidly to dark greyish-brown, even at the surface.

Line XR-916 shows the highest growth rate at 15°C and, after 15 days, reaches a colony size of 85 mm on MEA and a colony size of 70 mm on PDA. Under these conditions, the growth of line XR-909 on MEA amounts to 62 mm and on PDA to 52 mm. Line XR-913 shows a growth of 85 mm diameter on PDA and of 68 mm diameter on MEA. Line XR-917 shows the same growth on both media and reaches colony sizes of in each case 85 mm.

At 21°C, the growth of the newly obtained lines is poorer. After 15 days, line XR-916 reaches 46 mm on PDA and 51 mm on MEA. During this time, line XR-909 reaches 45 mm on PDA and 50 mm on MEA. Other lines have colony sizes of 85 mm on broth media at 21°C.

At 27°C, the newly obtained lines have ceased to grow, only line XR-913 reaches, after 15 days, a diameter of 52 mm on PDA and of 42 mm on MEA.

Some of the lines, such as XR-916, develop dark vesicles in the agar, which can be discerned under the microscope. The development of spores or conidia of the corresponding imperfect stage of the Xylariaceae in culture is observed very rarely.

*Fusarium* and *Verticillium* isolates show good growth at temperatures between 22 and 25°C and *Beauveria* isolates at 20°C (on mSeed medium).

### Composition of the media used:

| | |
|---|---|
| MEA: | malt extract 17 g/l, agar 15 g/l |
| PDA: | glucose 5 g/l, potato starch 20 g/l, agar 15 g/l |
| CMA: | cornmeal 50 g/l, agar 15 g/l |
| mSeed agar: | Pharmamedia 20 g/l, soya peptone 2 g/l, maltose 40 g/l, MgSO₄.7H2O 2 g/l, NaCl 2 g/l, CaCO₃ 3 g/l, agar 15 g/l |

### Description of the isolation and identification of novel 24-membered cycloocta-depsipeptides:

To rapidly identify the constituents, the methanolic mycelial extracts from the CBS fungal strains (CBS 111.75, CBS 267.30, CBS 445.89, CBS 446.89, CBS 447.89, CBS 448.89, CBS 449.89, CBS 450.89, CBS 499.80) and from the newly obtained lines or isolates (XR-909, XR-913, XR-916, XR-917, XR-21, etc.), and also from further fungal strains from the groups *Fusarium, Beauveria* and *Verticillium* were first analysed by means of LC-PDA-ESI-Q-TOF-MS

The detailed studies on the identification of the constituents were performed by means of LC-ESI=Q-TOF-MS/MS and -pseudoMS³ in aqueous and in deuterated solvents (H/D exchange experiments). Many known, but also novel, cyclic tetra-, hexa-, octa- and deca-depsipeptides in the mycelial extracts of the abovementioned fungal strains were identified by means of the abovementioned MS techniques.

Although the abovementioned CBS fungal strains have already been deposited in the CBS strain collection between 1930 and 1989, no published data are available as yet on the discovery of the cyclodepsipeptides from these strains. This is the first time that bassianolide from *Rosellinia* strains have been found.

To isolate the novel 24-membered cyclooctadepsipeptides for biological tests, the cell material was obtained from agar plates and extracted with methanol. The methanolic extracts were concentrated in vacuo until free from methanol, and then extracted repeatedly with ethyl acetate. The ethyl acetate extracts were concentrated in vacuo until free from ethyl acetate, then dried by lyophilization until free from water and subsequently separated by means of column chromatography (Silica Gel 60, n-hexane/ethyl acetate/methanol) into coarse fractions. Cyclooctadepsipeptides are obtained from the coarse fractions by means of preparative HPLC separation.

The detailed structure elucidation for novel main cyclooctadepsipeptides is performed by means of 1D- and 2D-NMR spectroscopy (¹H, ¹³C , DEPT, HMQC, HMBC, COSY, NOESY...).

From the abovementioned CBS fungal strains and from the newly obtained lines or isolates (XR-21, XR-909, XR-913, XR-916, XR-917), the novel 24-membered cyclooctadepsipeptides (PF1022-V, PF1022-W, XRB-C894, XRB-C942, XRB-C976, XRB-C1010, XRB-C1044, XRB-E922, XRB-E956, XRB-E990, XRB-E1024, XRB-S958, XRB-S992, XRB-S1026, XRB-S1060) were identified, partially isolated and tested (both as a mixture and individually).

Owing to the positive results of the *in-vitro* tests with *Trichinella* and *Nippostrongylus*, the use of the abovementioned novel 24-membered cyclooctadepsipeptides (in various isomeric forms), individually or as a mixture, is herewith patented as anthelmintics or endoparasiticides.

### Analytical data:

### 1. Working condition for the isolation and identification

### 1.1. Coarse separation of the extract of strain XR-916 by means of column chromatography:

| | |
|---|---|
| Sample size: | 5 g of dried ethyl acetate extract |
| Separating material: | 30 g,Silica Gel 60 (15 - 40 µm, Merck) |
| Column dimensions: | 20 cm × 2 cm |
| Flow rate: | 2 ml/min |
| Fraction volume: | 50 ml |

### Mobile phase:

| | |
|---|---|
| Fraction 1 - 2: | n-hexane/ethyl acetate: 2/1 |
| Fraction 3 - 4: | n-hexane/ethyl acetate: 1/1 |
| Fraction 5 - 6: | n-hexane/ethyl acetate: 1/2 |
| Fraction 7 - 8: | n-hexane/ethyl acetate: 1/3 |
| Fraction 9 - 10: | n-hexane/ethyl acetate: 1/4 |
| Fraction 11 - 12: | n-hexane/ethyl acetate: 1/9 |
| Fraction 13 - 14: | ethyl acetate |
| Fraction 15 - 16: | ethyl acetate/methanol: 98/2 |
| Fraction 17 - 18: | ethyl acetate/methanol: 95/5 |
| Fraction 19 - 20: | ethyl acetate/methanol: 90/10 |
| Fraction 21 - 22: | ethyl acetate/methanol: 85/15 |
| Fraction 23 - 26: | ethyl acetate/methanol: 80/20 |

### Constituents of the fractions:

| | |
|---|---|
| Fraction 2 - 4: | bassianolide, XRB-C894, -C942 and -C976, |
| | XRB-D- and -E cyclooctadepsipeptides, X R B-F cyclodecadepsipeptides |
| Fraction 5 - 7: | XRB=C942, XRB-C976, XRB-B cyclohexadepsipeptides |
| Fraction 8 - 16: | XRB-C976, -C1010 and -C1044, XRB-A linear peptides, XRB-B peptides |
| Fraction 17 - 25: | enniatin C, XRB-S958, XRB-S992, XRB-S1026, XRB-S1060 |

### 1.2. Group separation of the coarse fractions of strain XR-916 by means of HPLC:

| | |
|---|---|
| HPLC column: | Luna C5 (5 µm, 250 x 4.6 mm) |
| Eluent A: | water (0.1% HCOOH) |
| Eluent B: | methanol (0.1% HCOOH) |
| Eluent C: | acetonitrile (0.1% HCOOH) |

### HPLC condition:

| **Time** | **A** | **B** | **C** | **Curve** | **Flow rate** |
|---|---|---|---|---|---|
| 0 min | 25 | 65 | 10 | 1 | 1 ml/min |
| 33 min | 20 | 65 | 15 | 6 | 1 ml/min |
| 34 min | 5 | 65 | 30 | 1 | 1 ml/min |
| 38 min | 5 | 65 | 30 | 1 | 1 ml/min |
| 39 min | 25 | 65 | 10 | 1 | 1 ml/min |
| 45 min | 25 | 65 | 10 | 1 | 1 ml/min |
| Column temperature: | | 40°C | | | |
| Injection volume: | | 15 µl | | | |
| PDA detector: | | 210 to 400 nm | | | |
| Fraction: | | 20 sec / fr. x 120 fr. (wait time: 1.75 min) | | | |

### Results:

| | |
|---|---|
| Fraction with Rₜ 10 to 20 min: | cyclo- and linear depsipeptides of the XRB-S, -A, -B group |
| Fraction with Rₜ 20 to 35 min: | cyclodepsipeptides of the XRB-C, -D, -E and -F group |

### 1.3. Fine separation of the depsipeptides of strain XR-916 by means of HPLC

### 1.3.1. Separation condition for polar depsipeptides (fr. with Rₜ from 10 to 20 min):

| | |
|---|---|
| HPLC column: | Luna C5 (5 µm, 250 x 4.6 mm) |
| Eluent A: | water (0.1% HCOOH) |
| Eluent B: | acetonitrile (0.1% HCOOH) |
| HPLC condition: | isocratic (A / B : 33 / 67) |
| Running time: | 45 min |
| Flow rate: | 0.6 ml/min |
| Column temperature: | 40°C |
| Injection volume: | 10 µl |
| PDA detector: | 210 to 400 nm |
| Fractions: | 18 sec / fr. x 120 fr. (wait time: 15 min) |
| Rₜ from 19.8 to 20.5 min: | linear depsipeptide XRB-A767 (C₄₂H₆₁N₃O₁₀, [M⁺H]⁺ *m*/*z* 768.4435) |
| Rₜ from 21.3 to 22.5 min: | linear depsipeptide XRB-A733 (C₃₉H₆₃N₃O₁₀, [M+H]⁺ *m*/*z* 734.4592) |
| Rₜ from 23.1 to 24.9 min: | linear depsipeptide XRB-A699 |
| | (**C₃₆H₆₅N₃O₁₀**, [M+H]⁺ *m*/*z* 700.4748) |
| Rₜ from 31.8 to 32.7 min: | XRB-S992 |
| Rₜ from 33.3 to 35.4 min: | XRB-S958 |
| Rₜ from 37.8 to 40.2 min: | enniatin C (**C₃₆H₆₃N₃O₉**, [M+H]⁺ *m*/*z* 682.4643) |

### 1.3.2. Separation condition for cyclooctadepsipeptides (fr. with Rₜ from 20 to 35 min):

| | |
|---|---|
| HPLC column: | Luna C5 (5 µm, 250 x 4.6 mm) |
| Eluent A: | water (0.1 % HCOOH) |
| Eluent B: | acetonitrile (0.1 % HCOOH) |
| HPLC condition: | isocratic (A / B : 25 / 75) |
| Running time: | 25 min |
| Flow: | 1 ml/min |
| Column temperature: | 40°C |
| Injection volume: | 10 µl |
| PDA detector: | 210 to 400 nm |
| Fractions: | 18 sec / fr. x 120 fr. (wait time: 2 min) |
| Rₜ 15.8 to 16.5 min: | XRB-C1044 |
| Rₜ 17.3 to 18.3 min: | XRB-C1010 |
| Rₜ 19.3 to 20.8 min: | XRB-C976 |
| Rₜ 22 to 23.5 min: | XRB-C942 |

### 1.4. Conditions of the LC-MS, -MS/MS and -MS-H/D exchange experiments

### 1.4.1. Conditions of the LC-MS and -MS/MS experiments for strain XR-21

| | |
|---|---|
| HPLC column: | GeminiNX C18 (3 µm, 150 x 2 mm) |
| Eluent **A**: | water / acetonitrile: 9 / 1 (0.1% HCOOH) |
| Eluent **B**: | acetonitrile (0.1% HCOOH) |

### HPLC condition:

| **Time** | **A** | **B** | **Curve** | **Flow rate** |
|---|---|---|---|---|
| 0 min | 40 | 60 | 1 | 0.25 ml/min |
| 36 min | 20 | 80 | 6 | 0.25 ml/min |
| 37 min | 5 | 95 | 1 | 0.35 ml/min |
| 42 min | 5 | 95 | 1 | 0.35 ml/min |
| 43 min | 40 | 60 | 1 | 0.25 ml/min |
| 50 min | 40 | 60 | 1 | 0.25 ml/min |
| Column temperature: | | 40°C | | |
| Injection volume: | | 5 µl | | |
| PDA detector: | | 210 to 400 nm | | |
| MS detector: | | ESI-Q-TOF-MS (*full-scan, m*/*z* 130 to 2000) | | |

### 1.4.2. Conditions of the LC-MS and -MS/MS experiments for strain XR-916:

| | |
|---|---|
| HPLC column: | GeminiNX C18 (3 µm, 150 x 2 mm) |
| Eluent **A**: | water / acetonitrile: 9 / 1 (0.1% HCOOH) |
| Eluent **B**: | acetonitrile (0.1 % HCOOH) |

### HPLC condition:

| **Time** | **A** | **B** | **Curve** | **Flow rate** |
|---|---|---|---|---|
| 0 min | 40 | 60 | 1 | 0.2 ml/min |
| 105 min | 38 | 62 | 6 | 0.2 ml/min |
| 108 min | 25 | 75 | 6 | 0.35 ml/min |
| 140 min | 5 | 95 | 1 | 0.35 ml/min |
| 143 min | 5 | 95 | 1 | 0.35 ml/min |
| 144 min | 40 | 60 | 1 | 0.35 ml/min |
| 150 min | 40 | 60 | 1 | 0.35 ml/min |
| Column temperature: | | 40°C | | |
| Injection volume: | | 10 µl | | |
| PDA detector: | | 210 to 400 nm | | |
| MS detector: | | ESI-Q-TOF-MS (*full-scan, m*/*z* 130 to 2000) | | |

### 1.4.3. Conditions of the LC-MS-H/D exchange experiments:

| | |
|---|---|
| HPLC column: | GeminiNX C18 (3 µm, 150 x 2 mm) 18 |
| Eluent A: | D₂O (0.1% DCOOD) |
| Eluent B: | acetonitrile (0.1 % DCOOD) |

### HPLC condition:

| **Time** | **A** | **B** | **Curve** | **Flow rate** |
|---|---|---|---|---|
| 0 min | 35 | 65 | 1 | 0.2 ml/min |
| 80 min | 32 | 68 | 6 | 0.2 ml/min |
| 87 min | 5 | 95 | 1 | 0.2 ml/min |
| 92 min | 5 | 95 | 1 | 0.2 ml/min |
| 93 min | 35 | 65 | 1 | 0.2 ml/min |
| 100 min | 35 | 65 | 1 | 0.2 ml/min |
| Column temperature: | | 40°C | | |
| Injection volume: | | 10 µl | | |
| PDA detector: | | 210 to 400 nm | | |
| MS detector: | | ESI-Q-TOF-MS (*full-scan, m*/*z* 130 to 1600) | | |

### 2. Analytical data of the cyclooctadepsipeptides with 24 ring atoms

### 2.1. Analytical data of PF1022-A (1), PF1022-V (3) and PF1022-W (4)

### 2.1.1. PF1022-A (1)

| | |
|---|---|
| Empirical formula: | **C₅₂H₇₆N₄O₁₂** |
| Accurate mass: | 948.5460 |
| [M+H]⁺ (*m*/*z*): | found: 949.5540; calculated: 949.5538 |

ESI-MS/MS of *m*/*z* 949.6 (Rₜ = 23.6 min, *collision energy:* 25, 40, 75 V) [*m*/*z* (rel. int.)]: 100.1 (45), 140.1 (10), 172.1 (25), 182.1 (72), 200.1 (55), 216.1 (5), 230.1 (<5), 248.1 (23), 258.1 (100), 276.1 (55), 348.2 (18), 475.3 (19), 493.3 (5), 547.3 (<5), 595.4 (<5), 620.3 (<5), 674.4 (5), 750.4 (5), 768.5 (<5), 822.5 (<5), 949.6 (15).

### 2.1.2. PF1022-V (3)

| | |
|---|---|
| Empirical formula: | **C₅₁H₇₄N₄O₁₂** |
| Accurate mass: | 934.5303 |
| [M+H]⁺ (*m*/*z*): | found: 935.5390; calculated: 935.5382 |

ESI-MS/MS of *m*/*z* 935.5 (Rₜ = 23.4 min, *collision energy*: 25, 40, 75 V) [*m*/*z* (rel. int.)]: 86.1 (48), 100.1 (80), 140.1 (18), 154.1 (10), 172.1 (40), 182.1 (68), 200.1 (75), 218.1 (10), 230.1 (6), 248.1 (22), 258.1 (100), 276.1 (51), 285.1 (30), 313.2 (30), 348.2 (22), 388.2 (15), 445.3 (12), 460.3 (15), 475.3 (20), 587.4 (18), 662.4 (15), 738.4 (22), 919.5 (55), 937.5 (70).

### 2.1.3. PF1022-W (4)

| | |
|---|---|
| Empirical formula: | **C₅₁H₇₄N₄O₁₂** |
| Accurate mass: | 934.5303 |
| [M+H]⁺ (*m*/*z*): | found: 935.5390; calculated: 935.5382 |

ESI-MS/MS of *m*/*z* 935.5 (Rₜ = 24.4 min, *collision energy:* 25, 40, 75 V) [*m*/*z* (rel. int.)]: 100.1 (45), 112.1 (11), 140.1 (22), 172.1 (28), 182.1 (65), 200.1 (100), 218.1 (10), 248.1 (12), 258.1 (55), 276.1 (28), 334.2 (12), 348.2 (12), 434.3 (11), 461.3 (22), 475.3 (12), 533.4 (12), 551.4 (12), 660.4 (12), 708.5 (22), 935.5 (20).
ESI-MS/MS of *m*/*z* 935.5 (Rₜ = 26 min, *collision energy:* 25, 40, 75 V) [*m*/*z* (rel. int.)]: 100.1 (40), 140.1 (5), 158.1 (8), 172.1 (15), 182.1 (40), 200.1 (22), 216.1 (10), 248.1 (28), 258.1 (100), 276.1 (60), 348.2 (20), 433.3 (9), 461.3 (10), 475.3 (12), 533.4 (6), 632.4 (8), 660.4 (8), 709.4 (5), 736.4 (6), 760.4 (5), 808.4 (8), 907.5 (6), 935.5 (8).

### 2.2. Analytical data of bassianolide and of its novel derivatives (XRB-C894, XRB-C942, XRB-C976, XRB-C1010, XRB-C1044, XRB-D940, XRB-D974, XRB-E922, XRB-E956, XRB-E990, XRB-E1024, XRB-S958, XRB-S992, XRB-S1026, XRB-S1060) including their isomers

### 2.2.1. Bassianolide (2)

| | |
|---|---|
| Empirical formula: | **C₄₈H₈₄N₄O₁₂** |
| Accurate mass: | 908.6086 |
| [M+H]⁺ (*m*/*z*): | found: 909.6166; calculated: 909.6164 |

H/D exchange experiment: [M+D]⁺ *m*/*z* 910.63, [M+Na]⁺ *m*/*z* 931.6 (Rₜ = 49.7 min)
ESI-MS/MS of *m*/*z* 909.6 (Rₜ = 70 min, *collision energy:* 25 and 85 V) [*m*/*z* (rel. int.)]: 100.1 (55), 126.1 (15), 168.1 (11), 200.1 (13), 210.1 (100), 228.1 (65), 328.2 (8), 455.3 (18), 473.3 (8), 555.4 (5), 600.4 (5), 654.4 (<5), 682.4 (10), 700.4 (7), 782.4 (5), 909.6 (21).

### 2.2.2. XRB-C894 (5)

| | |
|---|---|
| Empirical formula: | **C₄₇H₈₂N₄O₁₂** |
| Accurate mass: | 894.5929 |
| [M+H]⁺ (*m*/*z*): | found: 895.5987; calculated: 895.6008 |

ESI-MS/MS of *m*/*z* 895.6 (Rₜ = 79.8 min, *collision energy:* 25 and 85 V) [*m*/*z* (rel. int.)]: 86.1 (5), 100.1 (48), 126.1 (8), 168.1 (5), 200.1 (8), 210.1 (100), 228.1 (38), 313.2 (<5), 328.2 (8), 413.3 (5), 441.3 (8), 455.3 (7), 541.3 (<5), 555.4 (<5), 640.4 (6), 668.4 (6), 686.4 (6), 768.4 (<5), 895.5 (10).

### 2.2.3. XRB-C942 (6)

| | |
|---|---|
| Empirical formula: | **C₅₁H₈₂N₄O₁₂** |
| Accurate mass: | 942.5929 |
| [M+H]⁺ (*m*/*z*): | found: 943.6023; calculated: 943.6008 |

H/D exchange experiment: [M+D]⁺ *m*/*z* 944.61, [M+Na]⁺ *m*/*z* 965.59 (Rₜ = 47.5 min)
ESI-MS/MS of *m*/*z* 943.6 (Rₜ = 67 min, *collision energy:* 25 and 85 V) [*m*/*z* (rel. int.)]: 100.1 (52), 126.1 (10), 134.1 (22), 168.1 (5), 200.1 (10), 210.1 (100), 228.1 (68), 244.1 (21), 262.1 (20), 328.2 (8), 455.3 (13), 473.3 (5), 489.3 (11), 507.3 (5), 555.4 (<5), 589.4 (<5), 634.4 (<5), 682.4 (5), 716.4 (8), 734.4 (6), 816.4 (<5), 943.6 (25).

### 2.2.4. XRB-C976(7)

| | |
|---|---|
| Empirical formula: | **C₅₄H₈₀N₄O₁₂** |
| Accurate mass: | 976.5773 |
| [M+H]⁺ (*m*/*z*): | found: 977.5874; calculated: 977.5851 |

H/D exchange experiment: [M+D]⁺ *m*/*z* 977.59, [M⁺Na]⁺ *m*/*z* 999.57 (Rₜ = 45.3 min)
ESI-MS/MS of *m*/*z* 977.6 (Rₜ = 64.5 min, *collision energy:* 25 and 85 V) [*m*/*z* (rel. int.)]: 100.1 (50), 126.1 (12), 134.1 (58), 168.1 (10), 200.1 (15), 210.1 (100), 228.1 (60), 244.1 (68), 262.1 (50), 328.2 (10), 362.2 (10), 455.3 (5), 489.3 (30), 507.3 (10), 589.4 (8), 716.4 (8), 750.4 (9), 850.4 (5), 977.6 (40).

### 2.2.5. XRB-C1011 (8)

| | |
|---|---|
| Empirical formula: | **C₅₇H₇₈N₄O₁₂** |

| | |
|---|---|
| Accurate mass: | 1010.5616 |
| [M+H]⁺ (*m*/*z*): | found: 1011.5710; calculated: 1011.5695 |

H/D exchange experiment: [M+Na]⁺ *m*/*z* 1033.58 (Rₜ = 43.3 min)
ESI-MS/MS of *m*/*z* 1011.6 (Rₜ = 61.8 min, *collision energy:* 25 and 85 V) [*m*/*z* (rel. int.)]: 100.1 (36), 134.1 (100), 168.1 (5), 200.1 (8), 210.1 (58), 228.1 (36), 244.1 (100), 262.1 (78), 328.2 (5), 362.2 (10), 489.3 (18), 507.3 (5), 523.3 (15), 589.4 (5), 623.4 (5), 668.4 (6), 722.4 (5), 750.4 (10), 768.4 (5), 1011.6 (18).

### 2.2.6. XRB-C1044 (9)

| | |
|---|---|
| Empirical formula: | **C₆₀H₇₆N₄O₁₂** |
| Accurate mass: | 1044.5460 |
| [M+H]⁺ (*m*/*z*): | found: 1045.5552; calculated: 1045.5538 |

H/D exchange experiment: [M+Na]⁺ *m*/*z* 1067.536 (Rₜ = 41.4 min)
ESI-MS/MS of *m*/*z* 1045.6 (Rₜ = 59.4 min, *collision energy:* 25 and 85 V) [*m*/*z* (rel. int.)]: 134.1 (60), 216.1 (9), 234.1 (16), 244.1 (100), 262.1 (70), 362.2 (17), 523.3 (12), 541.3 (9), 623.4 (5), 702.4 (6), 756.5 (7), 784.5 (7), 802.5 (8), 884.5 (5), 1045.6 (15).

### 2.2.7. XRB-E922 (10)

| | |
|---|---|
| Empirical formula: | **C₄₉H₈₆N₄O₁₂** |
| Accurate mass: | 922.6242 |
| [M+H]⁺ (*m*/*z*): | found: 923.6338; calculated: 923.6321 |

H/D exchange experiment: [M+D]⁺ *m*/*z* 924.64, [M+Na]⁺ *m*/*z* 945.61 (Rₜ = 64.4 min)
ESI-MS/MS of *m*/*z* 923.6 (Rₜ = 93.6 min, *collision energy*: 25, 45, 75 V) [*m*/*z* (rel. int.)]: 100.1 (62), 126.1 (12), 168.1 (5), 200.1 (10), 210.1 (100), 224.1 (33), 228.1 (50), 242.2 (19), 328.2 (5), 342.2 (<5), 455.3 (10), 469.3 (10), 487.3 (<5), 555.4 (<5), 569.4 (<5), 600.4 (<5), 614.4 (<5), 668.4 (<5), 682.4 (<5), 696.4 (7), 714.4 (6), 796.5 (5), 923.6 (23).

### 2.2.8. XRB-E956 (11)

| | |
|---|---|
| Empirical formula: | **C₅₂H₈₄N₄O₁₂** |
| Accurate mass: | 956.6086 |
| [M+H]⁺ (*m*/*z*): | found: 957.6178; calculated: 957.6164 |

H/D exchange experiment: [M+Na]⁺ *m*/*z* 979.6 (Rₜ = 61.5 min)
ESI-MS/MS of *m*/*z* 957.6 (Rₜ = 88 min, *collision energy:* 25, 45, 75 V) [*m*/*z* (rel. int.)]: 100.1 (60), 126.1 (13), 134.1 (25), 168.1 (10), 200.1 (10), 210.1 (100), 224.1 (<5), 228.1 (50), 258.1 (19), 276.1 (12), 328.2 (7), 342.2 (<5), 455.3 (12), 473.3 (<5), 503.3 (7), 521.3 (<5), 569.4 (<5), 682.4 (<5), 730.4 (6), 748.4 (5), 830.4 (<5), 957.6 (23).
ESI-MS/MS of *m*/*z* 957.6 (Rₜ = 89.4 min, *collision energy:* 25, 45, 75 V) [*m*/*z* (rel. int.)]: 100.1 (85), 126.1 (13), 134.1 (35), 168.1 (8), 200.1 (11), 210.1 (100), 224.1 (45), 228.1 (60), 242.1 (25), 244.1 (28), 262.1 (21), 328.2 (<5), 342.2 (5), 455.3 (10), 469.3 (10), 489.3 (8), 503.3 (<5), 521.3 (<5), 569.4 (<5), 682.4 (<5), 714.4 (5), 730.4 (7), 748.4 (5), 830.4 (5), 957.6 (23).
ESI-MS/MS of *m*/*z* 957.6 (Rₜ = 98.1 min, *collision energy:* 25, 45, 75 V) [*m*/*z* (rel. int.)]: 100.1 (55), 126.1 (33), 134.1 (40), 168.1 (8), 200.1 (11), 210.1 (100), 224.1 (25), 228.1 (51), 242.1 (25), 244.1 (35), 262.1 (28), 328.2 (8), 342.2 (5), 362.2 (<5), 455.3 (8), 469.3 (12), 489.3 (10), 503.3 (<5), 521.3 (<5), 569.4 (<5), 648.4 (<5), 696.4 (<5), 716.4 (8), 730.4 (7), 748.4 (5), 830.4 (8), 957.6 (33).

### 2.2.9. XRB-E990 (12)

| | |
|---|---|
| Empirical formula: | **C₅₅H₈₂N₄O₁₂** |
| Accurate mass: | 990.5929 |
| [M+H]⁺ (*m*/*z*): | found: 991.6018; calculated: 991.6008 |

H/D exchange experiment: [M+Na]⁺ *m*/*z* 1013.58 (Rₜ = 57.9 min)
ESI-MS/MS of *m*/*z* 991.6 (Rₜ = 85.6 min, *collision energy:* 25 and 85 V) [*m*/*z* (rel. int.)]: 100.1 (88), 126.1 (15), 134.1(100), 168.1 (8), 200.1 (19), 210.1(92), 224.1(65), 228.1 (55), 242.1 (32), 244.1 (78), 258.1 (18), 262.1 (58), 276.1 (15), 328.2 (5), 342.2 (5), 362.2 (8), 376.2 (5), 489.3 (21), 503.3 (22), 521.3 (8), 603.4 (8), 730.4 (9), 764.4 (8), 782.4 (6), 864.4 (6), 991.6 (60).
ESI-MS/MS of *m*/*z* 991.6 (Rₜ = 93.4 min, *collision energy:* 25 and 85 V) [*m*/*z* (rel. int.)]: 100.1 (32), 126.1 (45), 134.1 (100), 168.1 (8), 200.1 (8), 210.1 (60), 224.1 (55), 228.1 (35), 242.1 (45), 244.1 (70), 262.1 (58), 342.2 (6), 362.2 (9), 489.3 (22), 503.3 (18), 589.4 (8), 648.4 (8), 730.4 (9), 764.4 (8), 782.4 (6), 864.4 (6), 991.6 (55).

### 2.2.10. XRB-E1024 (13)

| | |
|---|---|
| Empirical formula: | **C₅₈H₈₀N₄O₁₂** |
| Accurate mass: | 1024.5773 |
| [M+H]⁺ (*m*/*z*): | found: 1025.5865; calculated: 1025.5851 |

ESI-MS/MS of *m*/*z* 1025.6 (Rₜ = 81.6 min, *collision energy:* 25 and 85 V) [*m*/*z* (rel. int.)]: 100.1 (30), 134.1 (100), 210.1 (35), 224.1 (20), 228.1 (20), 234.1 (10), 242.1 (25), 244.1 (80), 258.1 (21), 262.1 (55), 276.1 (15), 362.2 (5), 376.2 (5), 489.3 (9), 503.3 (8), 523.3 (10), 537.3 (8), 603.4 (<5), 764.4 (15), 782.4 (6), 816.4 (5), 864.4 (6), 898.5 (6), 1025.6 (48).

### 2.2.11. XRB-S958 (14)

| | |
|---|---|
| Empirical formula: | **C₄₇H₈₂N₄O₁₄S** |
| Accurate mass: | 958.5548 |
| [M+H]⁺ (*m*/*z*): | found: 959.5628; calculated: 959.5627 (FT-ICR-MS) |

H/D exchange experiment: [M+Na]⁺ *m*/*z* 981.54 (Rₜ = 16.0 min)
ESI-MS/MS of *m*/*z* 959.6 (Rₜ = 21.2 min, *collision energy:* 25, 45, 75 V) [*m*/*z* (rel. int.)]: 70.1 (5), 100.1 (35), 126.1 (5), 150.1 (8), 168.1 (8), 180.1 (25), 200.1 (18), 210.1 (100), 228.1 (53), 260.1 (11), 278.1 (14), 328.2 (9), 378.2 (5), 455.3 (5), 505.3 (7), 523.3 (5), 555.4 (<5), 605.4 (<5), 732.4 (<5), 750.4 (<5), 832.5 (<5), 959.6 (12).
ESI-pseudo-MS³ of *m*/*z* 150.1 [*m*/*z* (rel. int.)]: 54 (4), 55 (8), 68 (2), 70.1 (69), 71.1 (17), 801 (2), 121.1(2).

### 2.2.12. XRB-S992 (15)

| | |
|---|---|
| Empirical formula: | **C₅₀H₈₀N₄O₁₄S** |
| Accurate mass: | 992.5392 |
| [M+H]⁺ (*m*/*z*): | found: 993.5480; calculated: 993.5470 |

H/D exchange experiment: [M+Na]⁺ *m*/*z* 1015.53 (Rₜ = 14.9 min)
ESI-MS/MS of *m*/*z* 993.5 (Rₜ = 19.9 min, *collision energy:* 25, 45, 75 V) [*m*/*z* (rel. int.)]: 70.1 (8), 100.1 (40), 126.1 (5), 134.1 (18), 150.1 (15), 168.1 (8), 180.1 (31), 200.1 (20), 210.1 (100), 228.1 (62), 244.1 (36), 260.1 (18), 262.1 (19), 278.1 (25), 328.2 (12), 360.3 (5), 455.3 (8), 505.3 (9), 539.3 (8), 605.4 (<5), 650.4 (5), 732.4 (<5), 766.4 (<5), 866.5 (<5), 993.6 (30).
ESI-MS/MS of *m*/*z* 993.5 (Rₜ = 21.4 min, *collision energy:* 25, 45, 75 V) *[m*/*z* (rel. int.)]: 70.1 (5), 100.1 (25), 126.1 (8), 134.1 (25), 150.1 (12), 168.1 (5), 180.1 (35), 200.1(18), 210.1 (100), 228 (48), 244.1 (19), 260.1 (15), 262.1 (22), 278.1 (21), 328.2 (8), 360.3 (5), 378.2 (8), 455.3 (5), 505.3 (9), 784.4 (5), 866.5 (5), 993.6 (18).

### 2.2.13. XRB-S1026 (16)

| | |
|---|---|
| Empirical formula: | **C₅₃H₇₈N₄O₁₄S** |
| Accurate mass: | 1026.5235 |
| [M+H]⁺ *(m*/*z):* | found: 1027.5321; calculated: 1027.5314 |

H/D exchange experiment: [M+Na]⁺ *m*/*z* 1049.51 (Rₜ = 14.0 min)
ESI-MS/MS of *m*/*z* 1027.5 (Rₜ = 18.7 min, *collision energy:* 25, 45, 75 V) [*m*/*z* (rel. int.)]: 70.1 (7), 100.1 (30), 117.1 (5), 134.1 (35), 150.1 (10), 168.1 (5), 180.1 (30), 200.1 (15), 210.1 (40), 228 (40), 234.1 (20), 244.1 (100), 260.1 (18), 262.1 (41), 278.1 (24), 328.2 (10), 362.3 (12), 378.2 (9), 523.3 (11), 539 (8), 639.4 (6), 684.4 (5), 784.4 (5), 900.5 (8), 1027.6 (28).
ESI-MS/MS of *m*/*z* 1027.5 (Rₜ = 20 min, *collision energy:* 25, 45, 75 V) *[m*/*z* (rel. int.)]: 70.1 (10), 100.1 (22), 134.1 (65), 150.1 (12), 168.1 (6), 180.1 (45), 200.1 (13), 210.1 (78), 228 (48), 234.1 (19), 244.1 (100), 260.1 (15), 262.1 (68), 278.1 (38), 328.2 (5), 362.2 (15), 378.2 (9), 439.3 (5), 457.3 (5), 489.3 (11), 505.3 (5), 523.3 (5), 539.3 (15), 639.4 (5), 684.4 (5), 750.4 (8), 768.4 (5), 784.4 (<5), 800.4 (5), 866.4 (5), 900.5 (8), 1027.6 (28).

### 2.2.14. XRB-S1060 (17)

| | |
|---|---|
| Empirical formula: | **C₅₆H₇₆N₄O₁₄S** |
| Accurate mass: | 1060.5079 |
| [M+H]⁺ *(m*/*z):* | found: 1061.5165; calculated: 1061.5157 |

H/D exchange experiment: [M+Na]⁺ *m*/*z* 1083.50 (Rₜ = 13.2 min)
ESI-MS/MS of *m*/*z* 1061.5 (Rₜ = 18.9 min, *collision energy:* 25, 45, 85 V) [*m*/*z* (rel. int.)]: 134.1 (58), 150.1 (13), 180.1 (27), 234.1 (35), 244.1 (100), 260.1 (15), 262.1 (78), 278.1 (13), 362.2 (15), 523.3 (9), 539.3 (18), 557.3 (15), 639.4 (9), 772.4 (8), 818.4 (8), 1061.6 (22).

## Claims

1. 24-membered cyclooctadepsipeptides PF1022-V, PF1022-W, XRB-C894, XRB-C942, XRB-C976, XRB-C1010, XRB-C1044, XRB-E922, XRB-E956, XRB-E990, XRB-E1024, XRB-S958, XRB-S992, XRB-S1026, XRB-S1060 having the formulas:

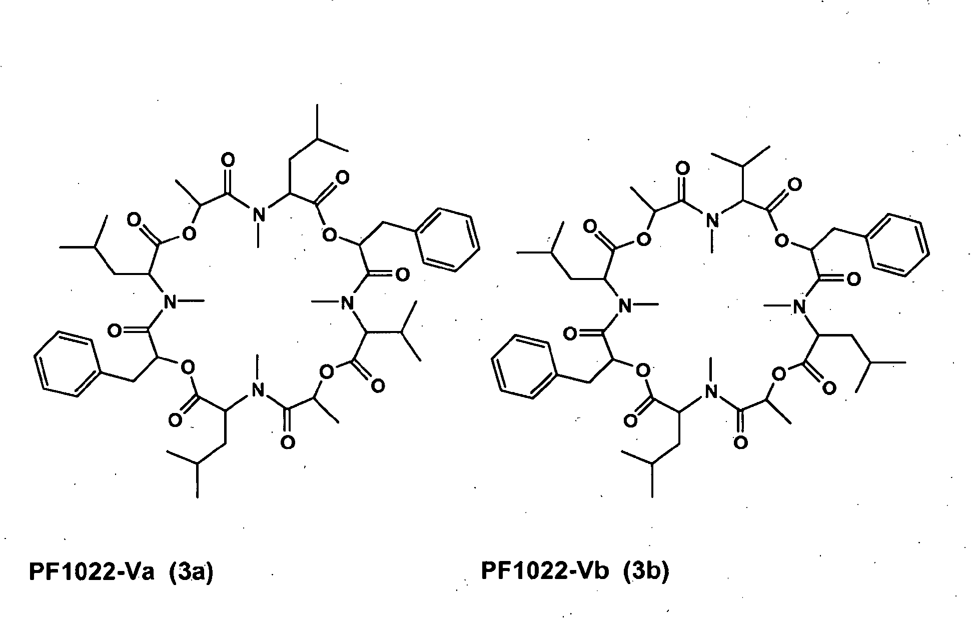

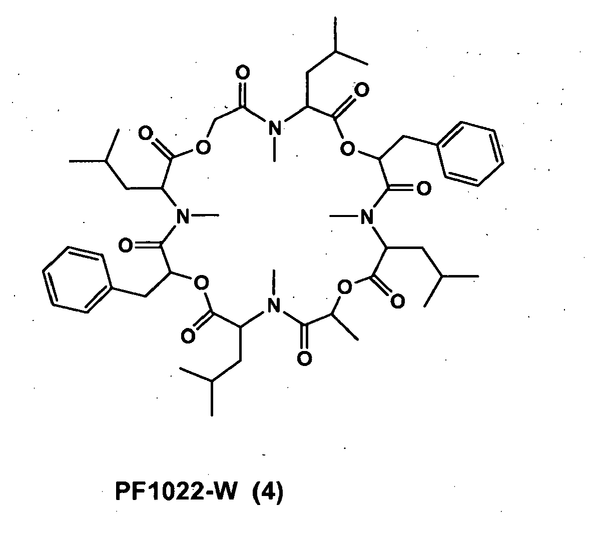

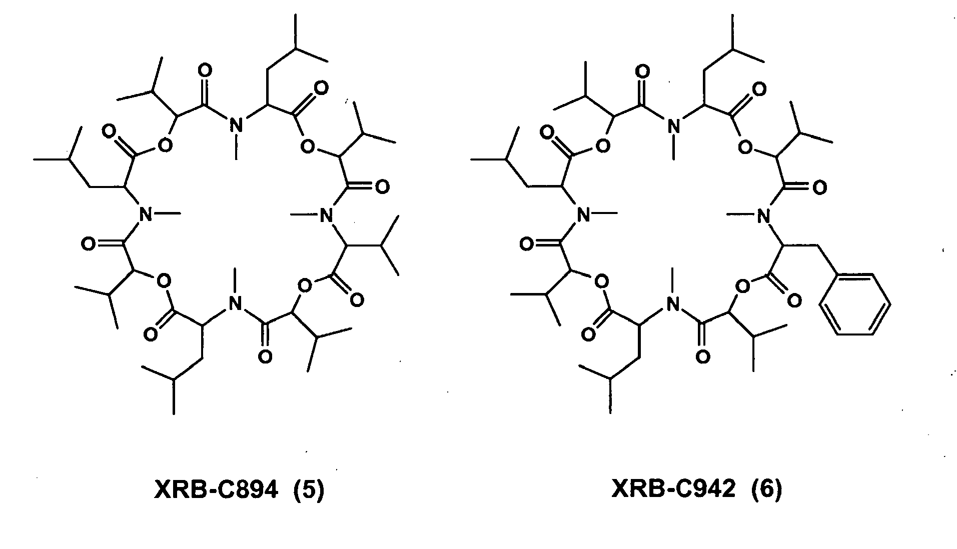

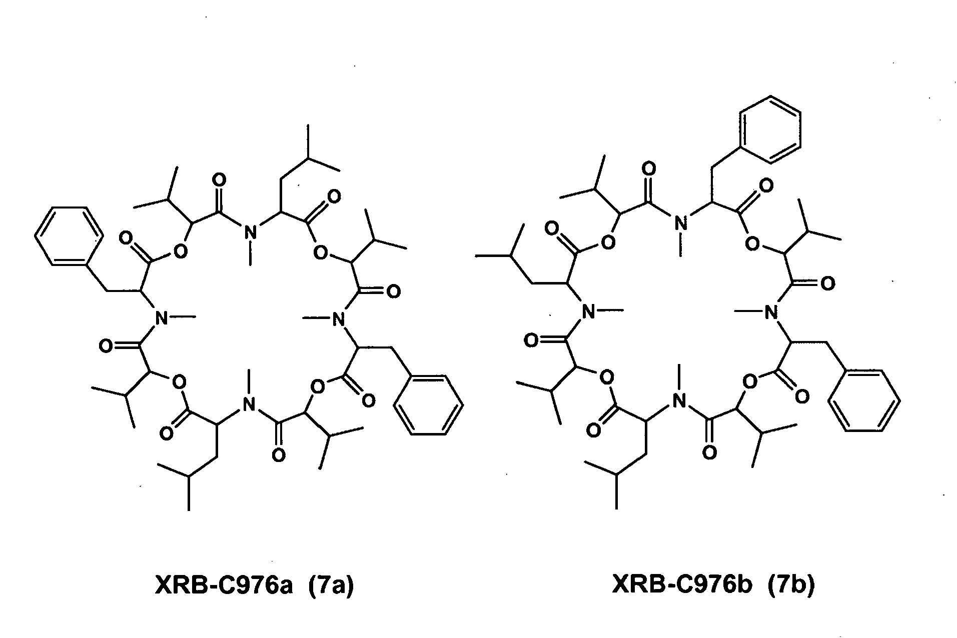

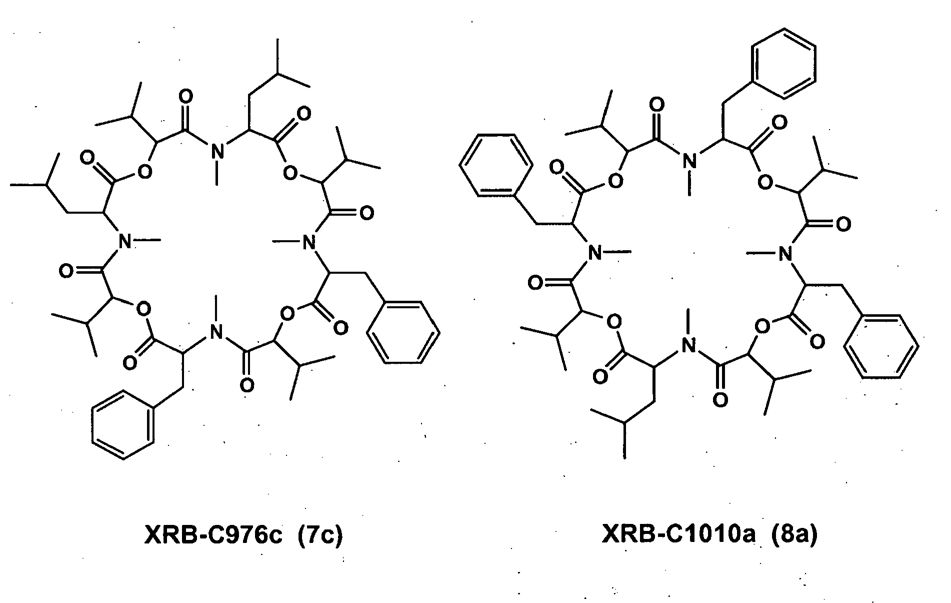

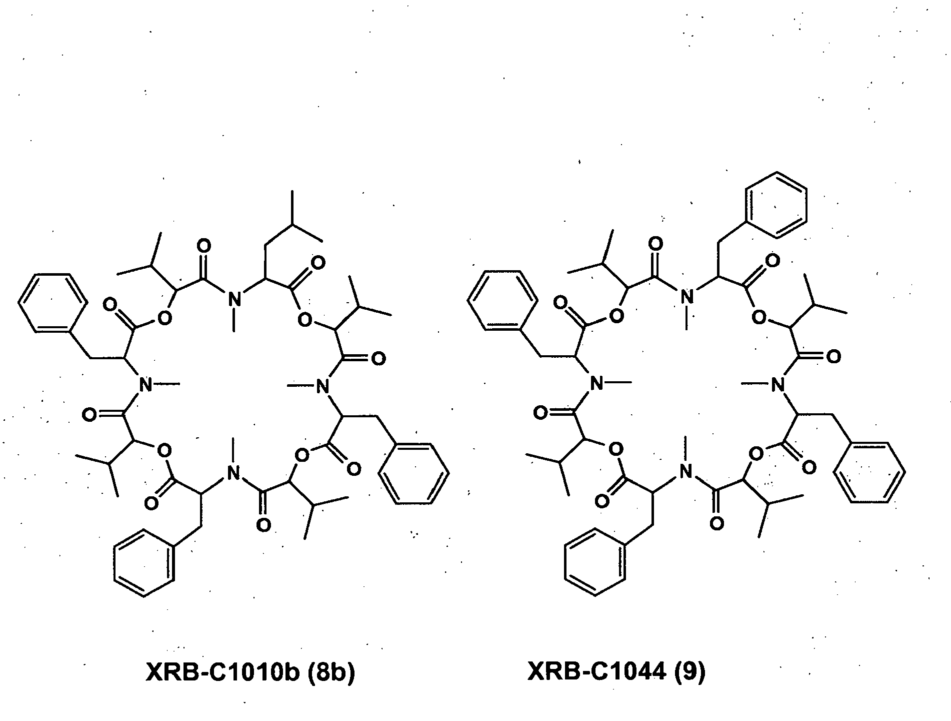

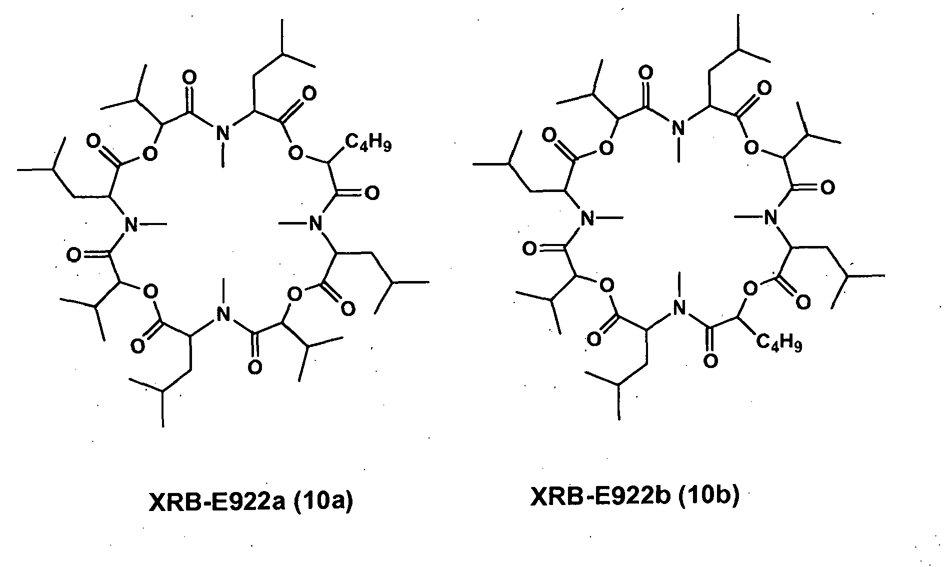

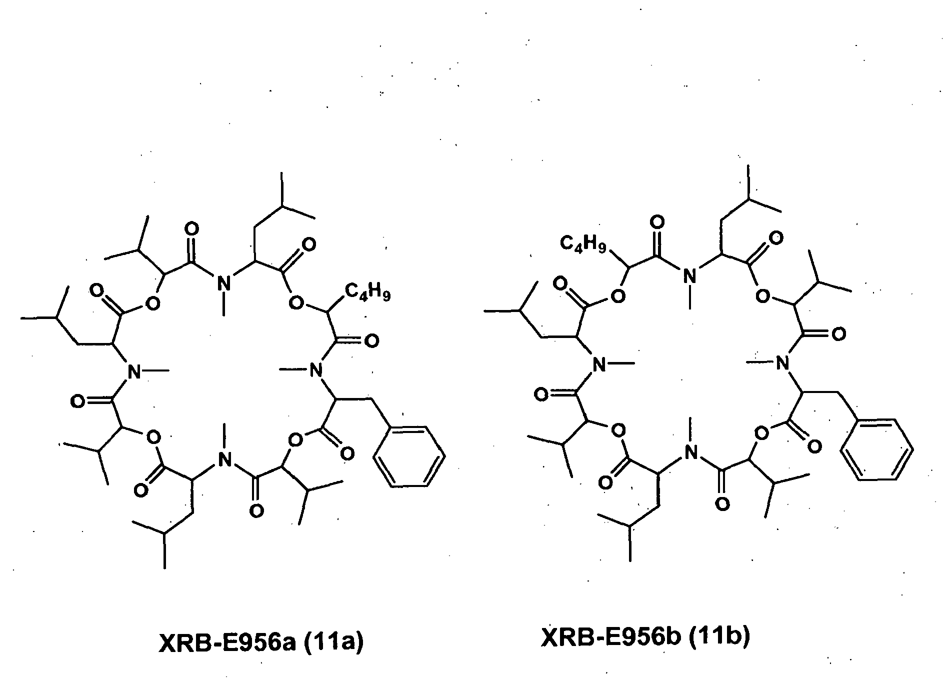

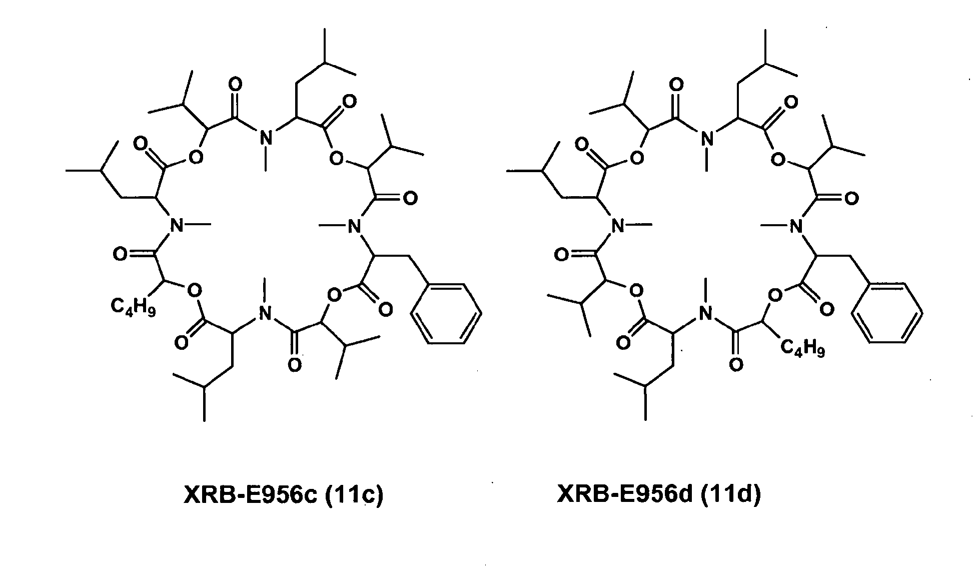

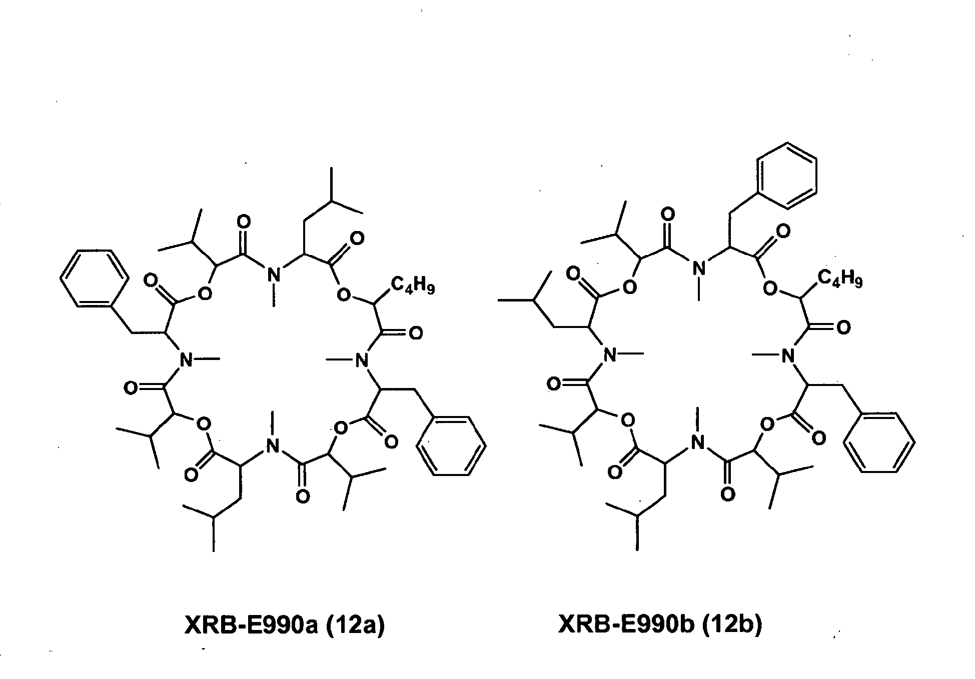

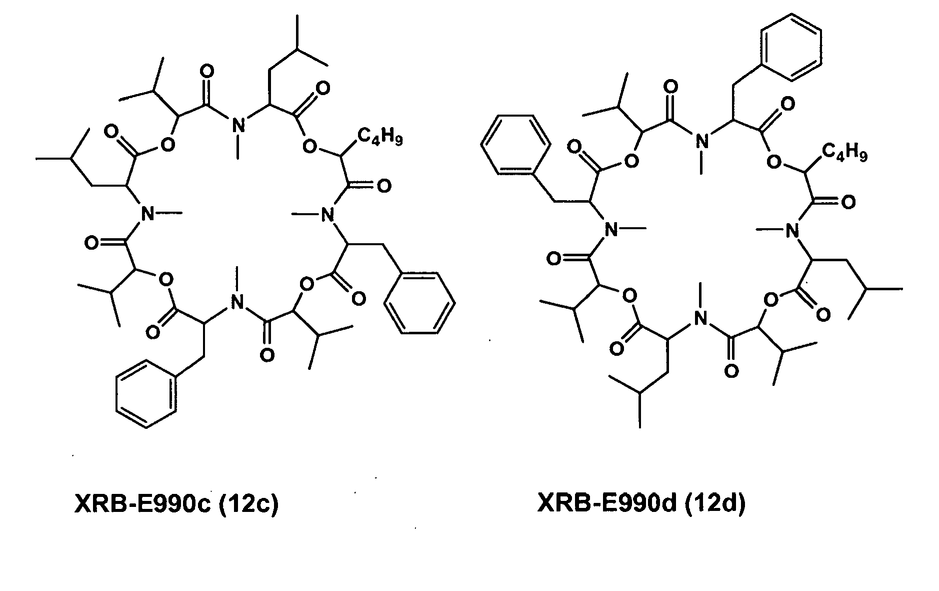

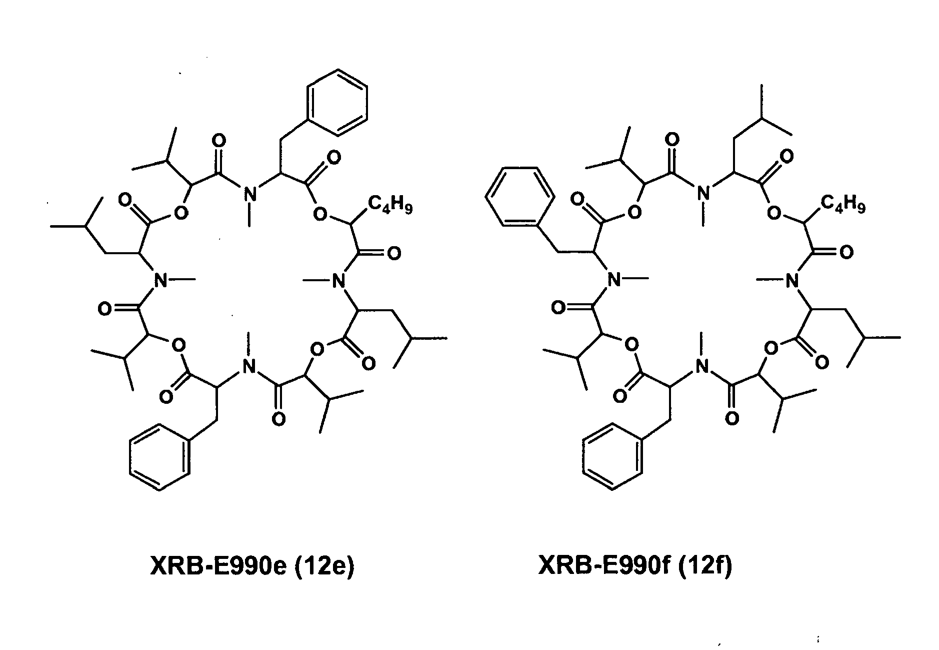

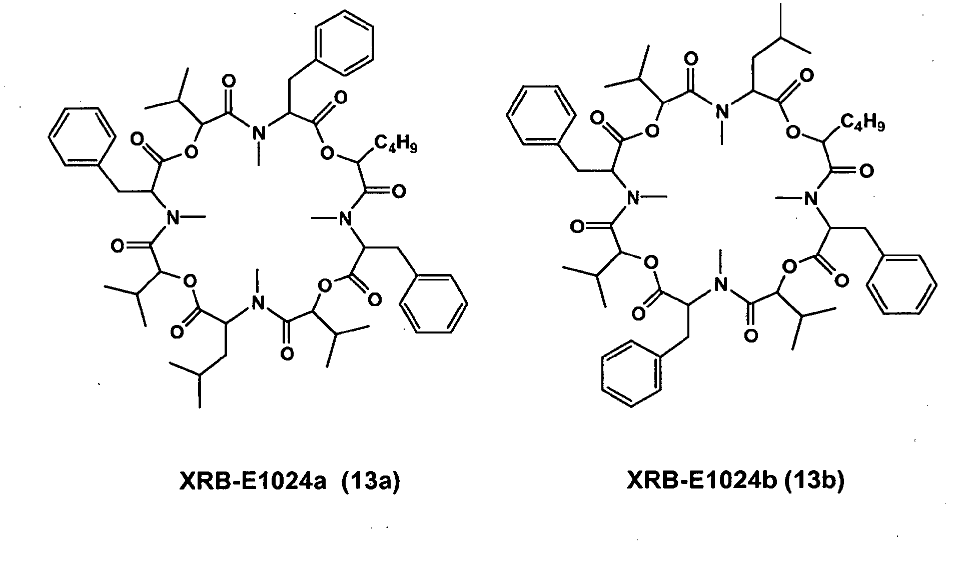

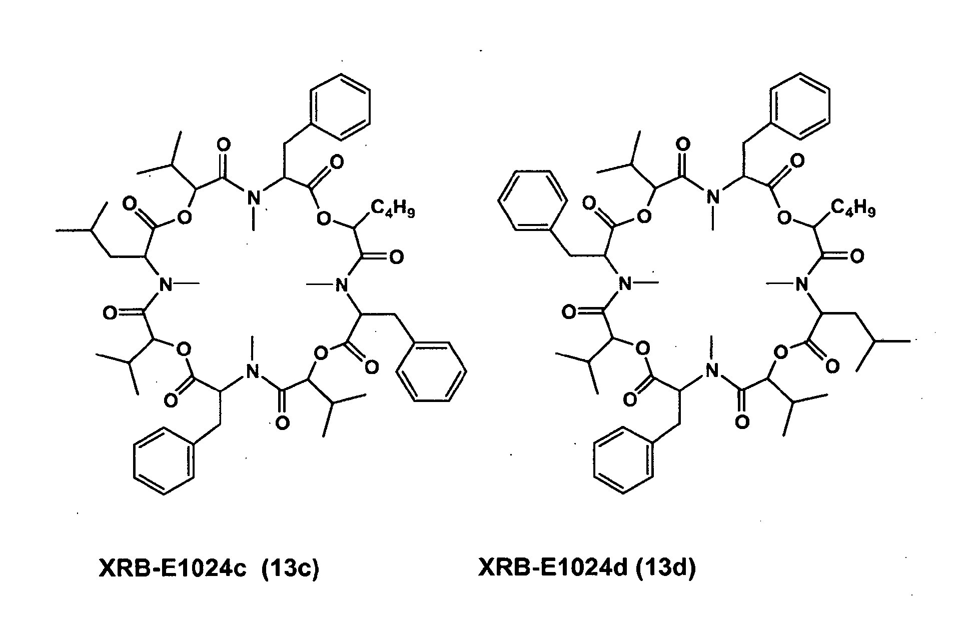

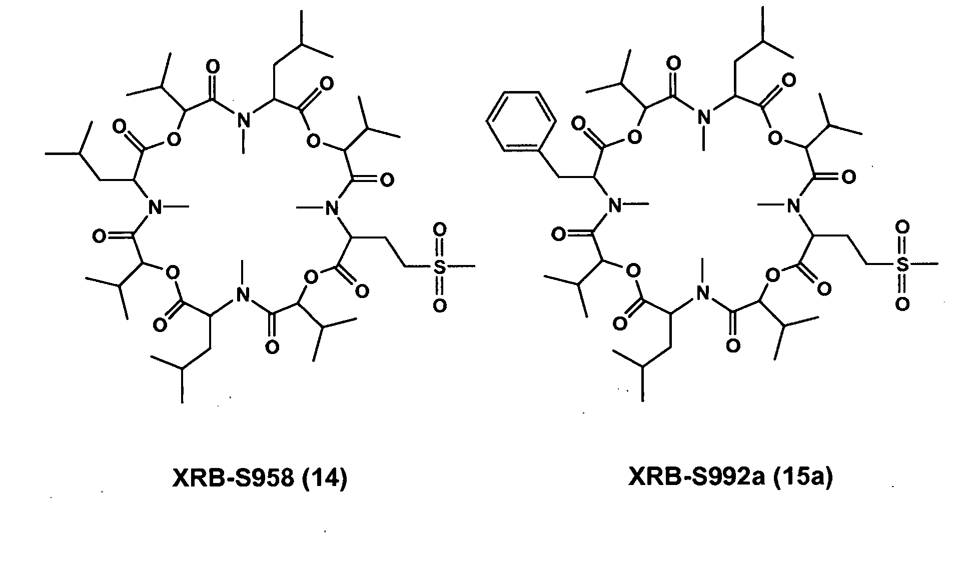

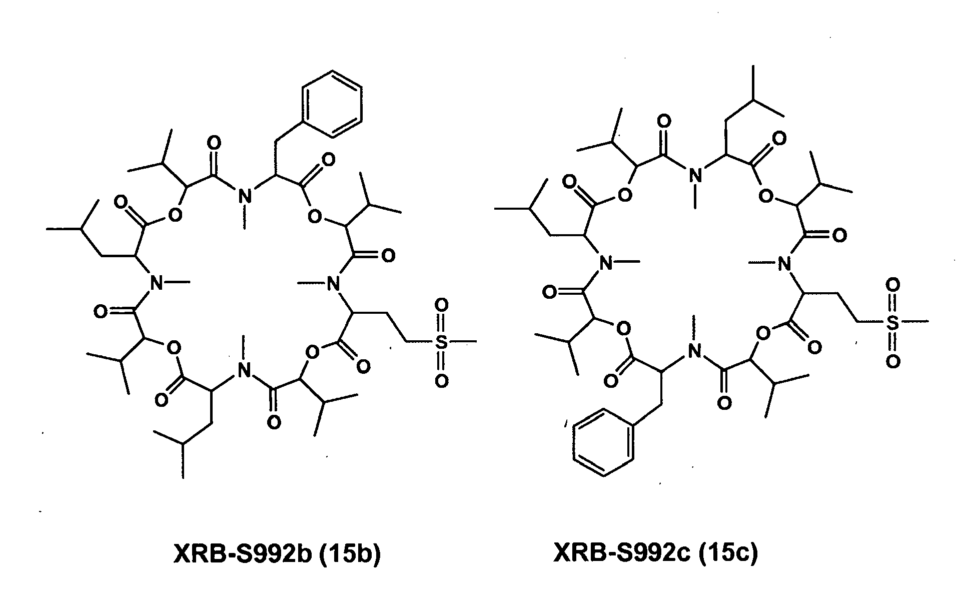

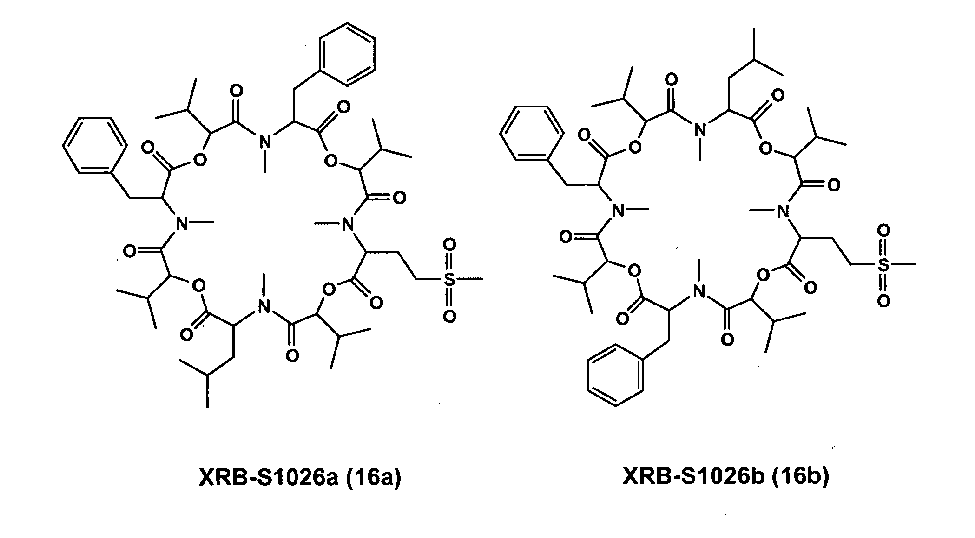

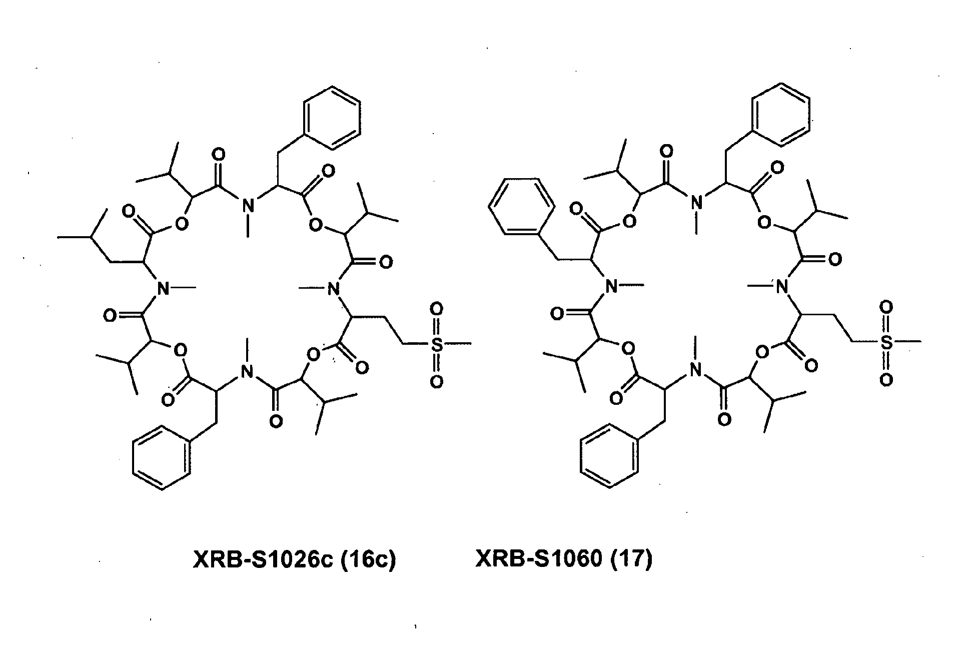

2. Cyclooctadepsipeptides according to Claim 1 individually or as a mixture with bassianolide for use as anthelmintics or endoparasiticide.

## Patentansprüche

1. 24-gliedrige Cyclooctadepsipeptide (PF1022-V, PF1022-W, XRB-C894, XRB-C942, XRB-C976, XRB-C1010, XRB-C1044, XRB-E922, XRB-E956, XRB-E990, XRB-E1024, XRB-S958, XRB-S992, XRB-S1026, XRB-S1060) mit den folgenden Formeln:

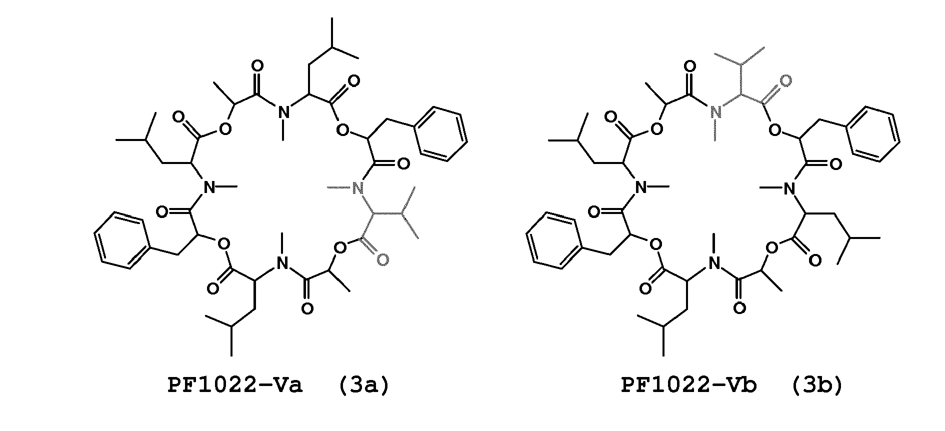

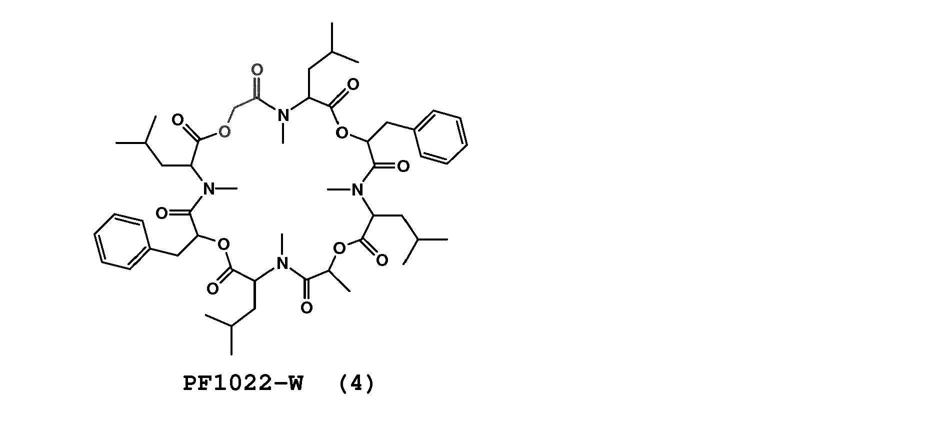

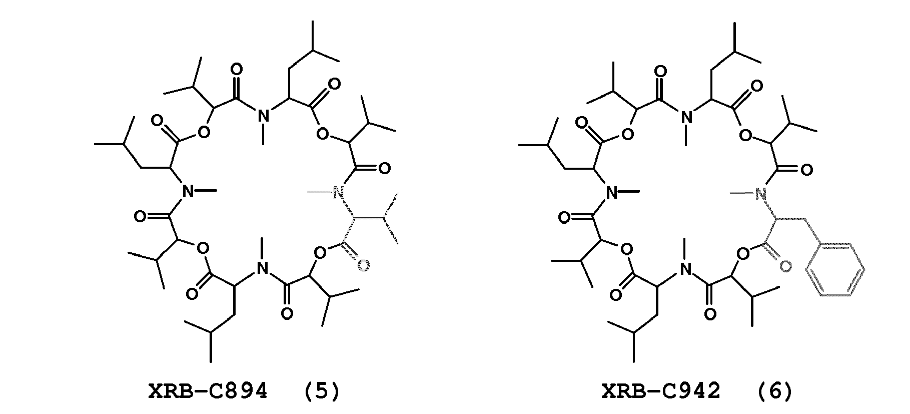

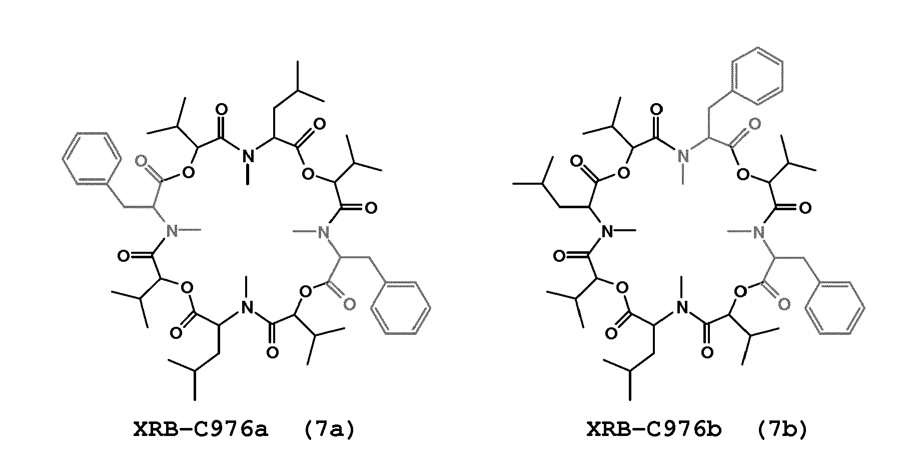

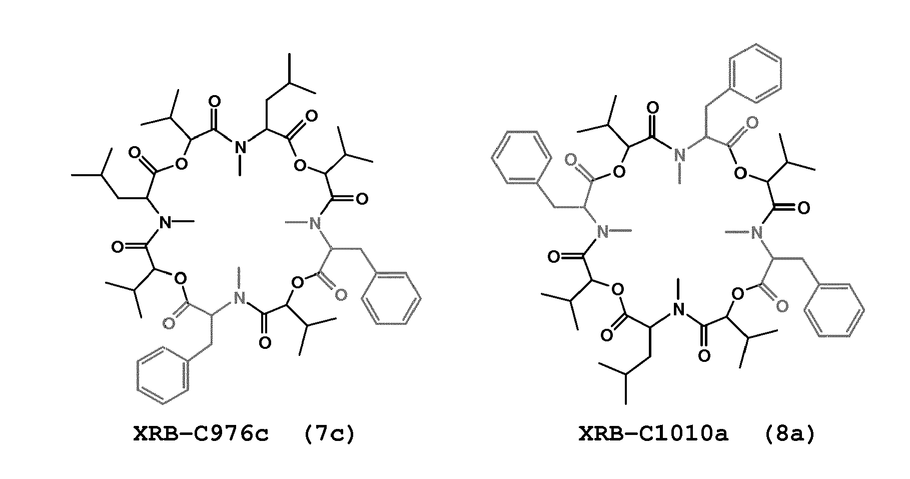

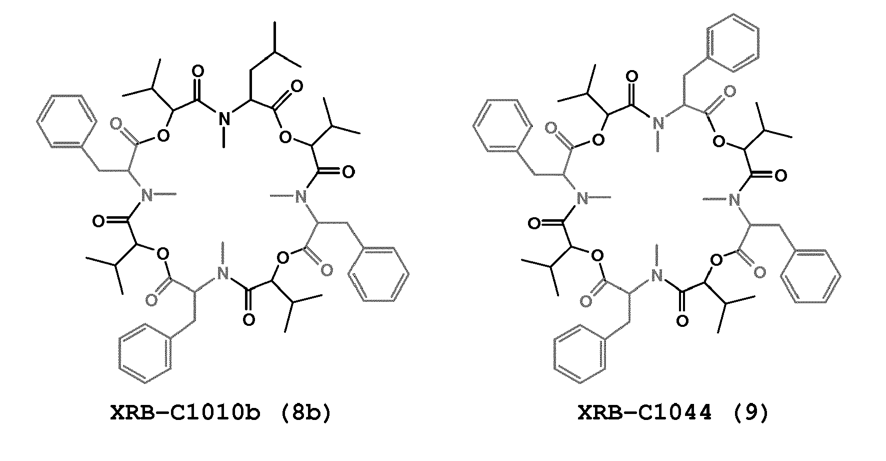

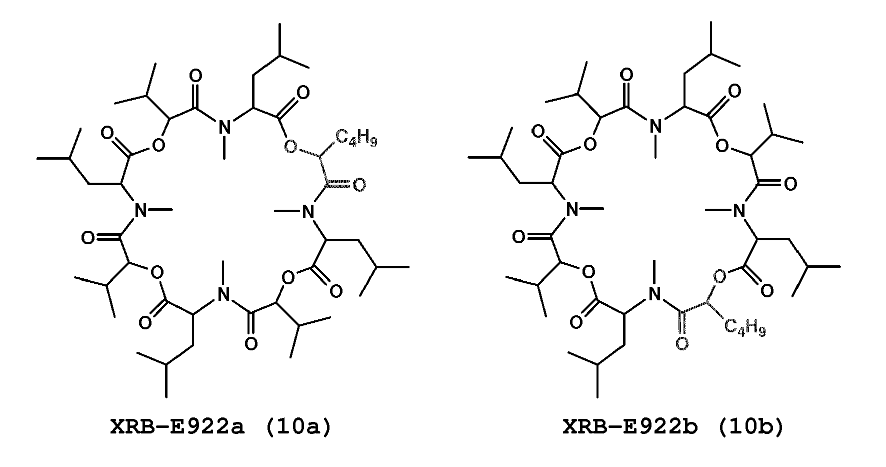

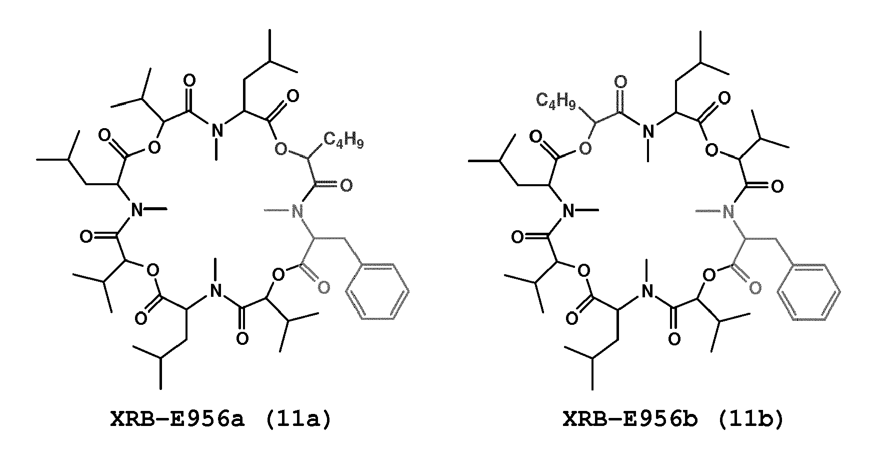

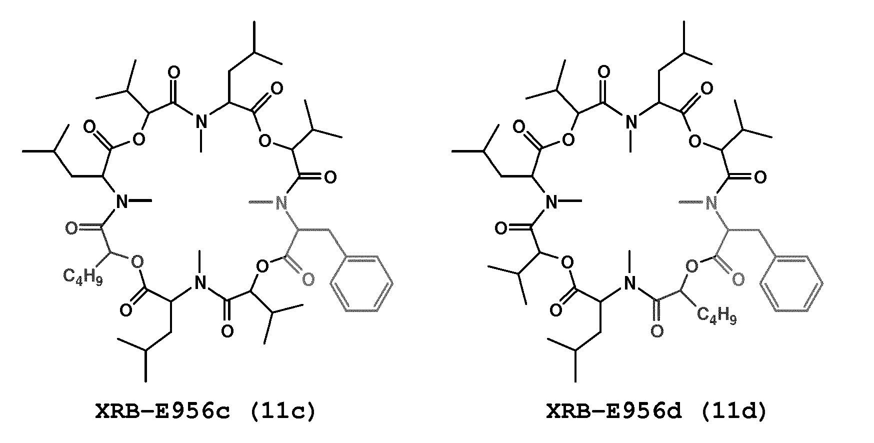

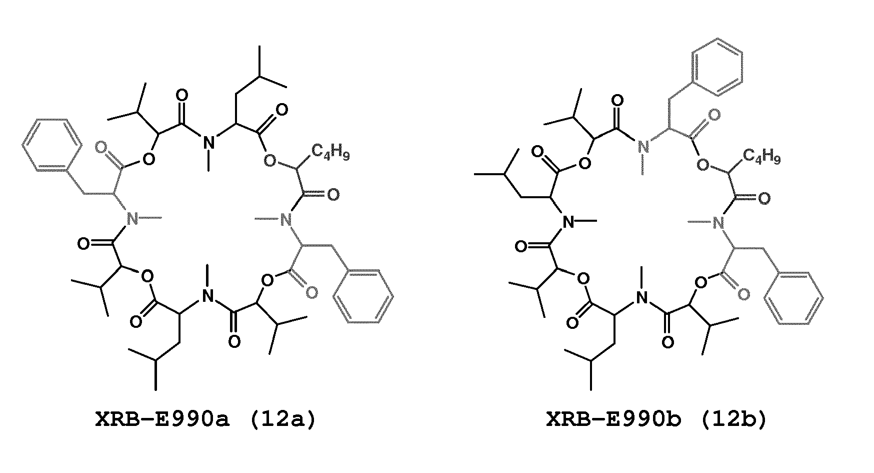

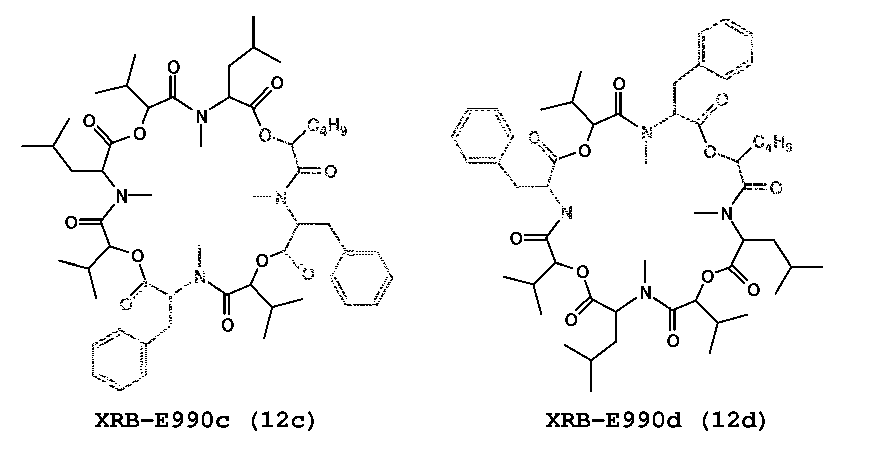

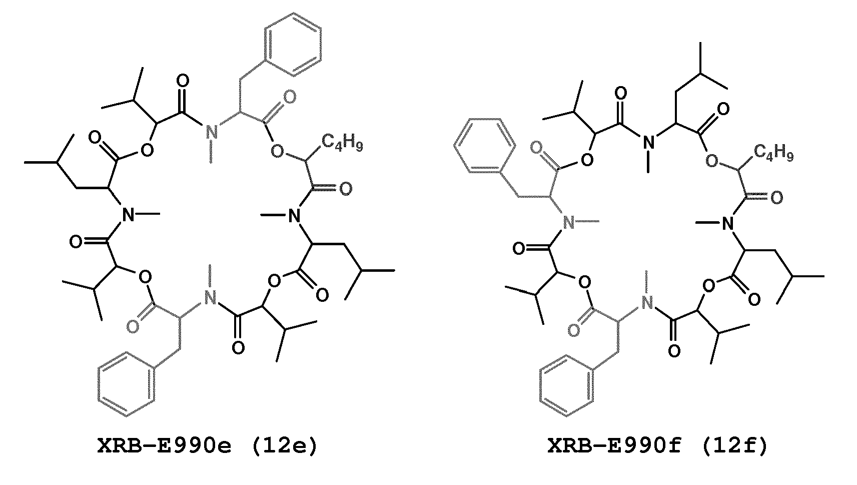

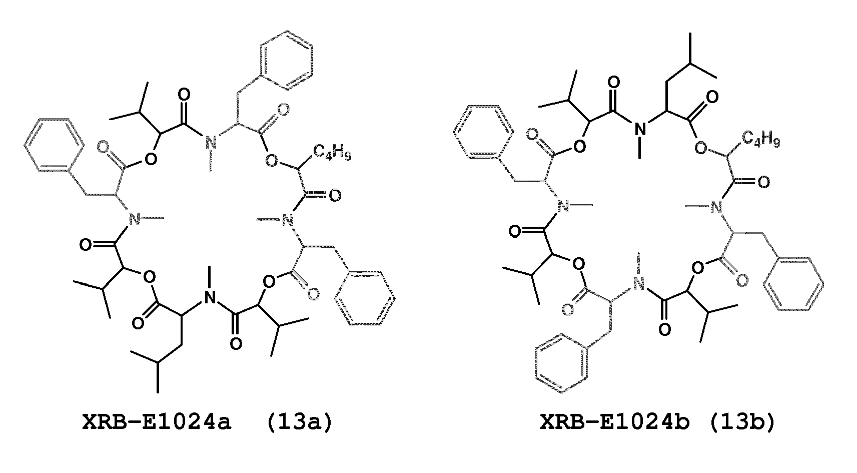

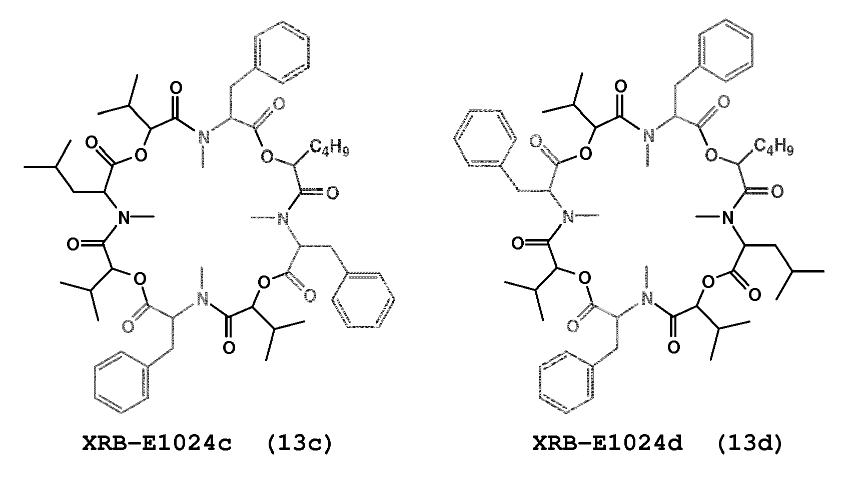

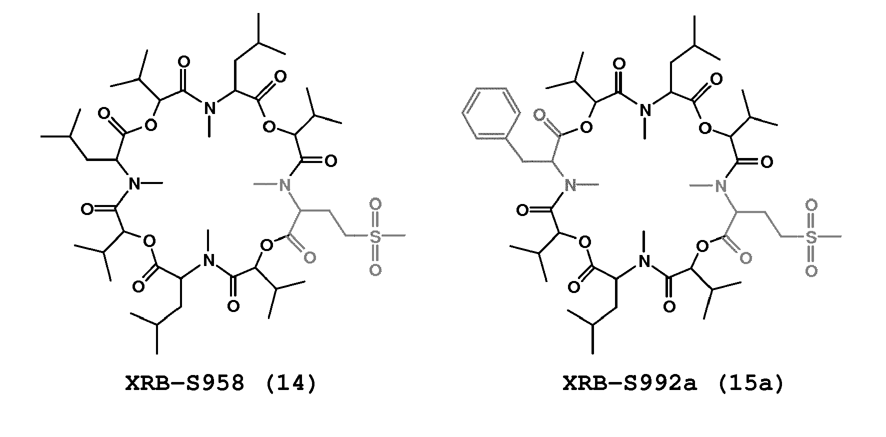

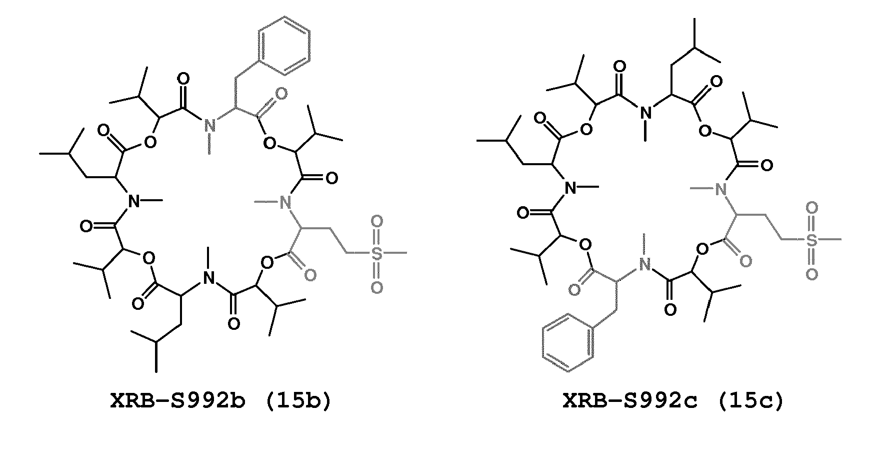

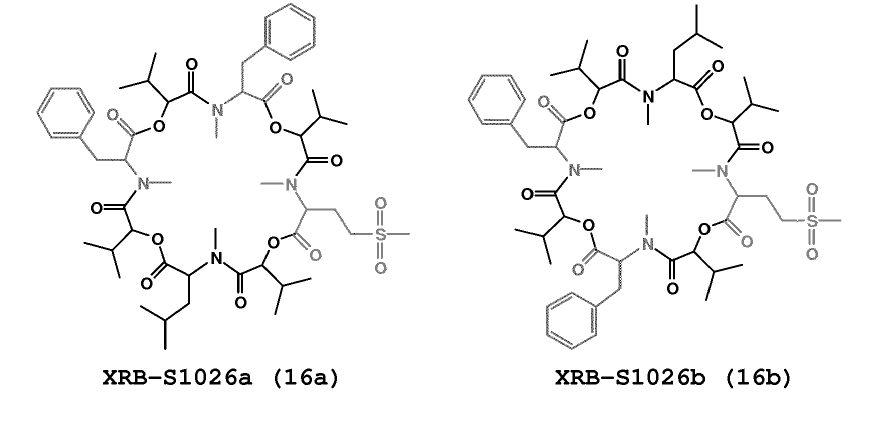

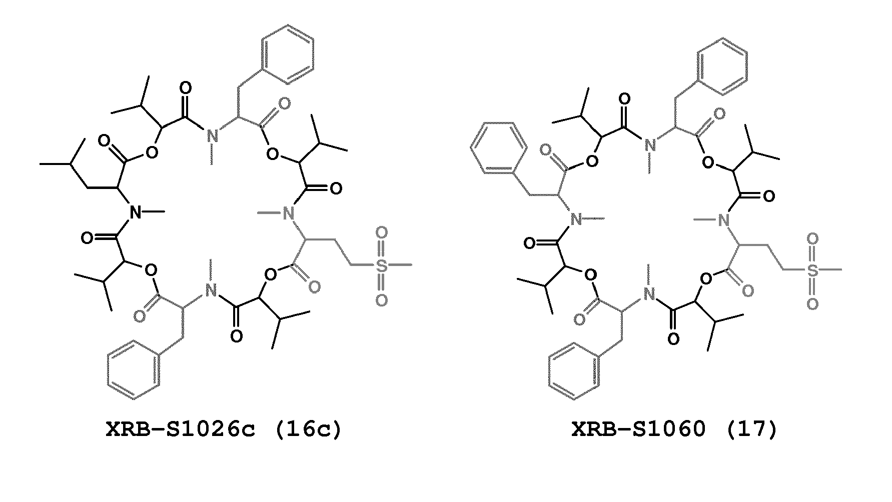

2. Verwendung der Cyclooctadepsipeptide gemäß Anspruch 1 im Einzelnen oder im Gemisch mit Bassianolid als Anthelminthika bzw. Endoparasitizide.

## Revendications

1. Cyclooctadepsipeptides à 24 chaînons PF1022-V, PF1022-W, XRB-C894, XRB-C942, XRB-C976, XRB-C1010, XRB-C1044, XRB-E922, XRB-E956, XRB-E990, XRB-E1024, XRB-S958, XRB-S992, XRB-S1026, XRB-S1060 répondant aux formules :

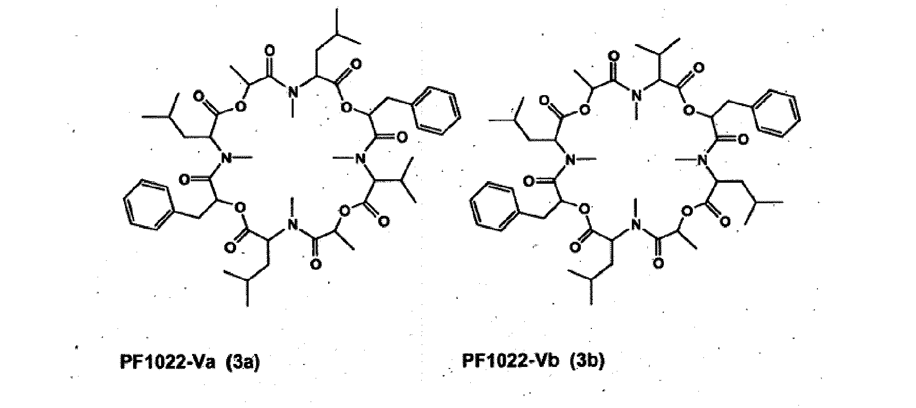

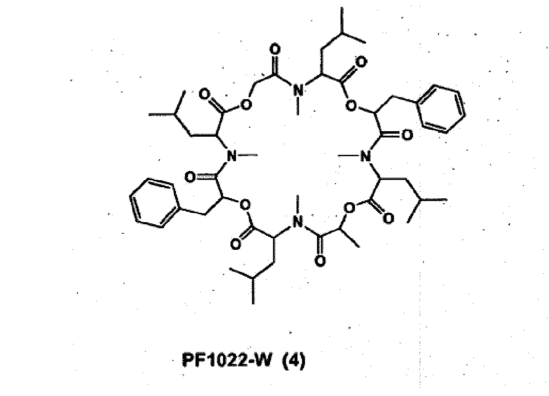

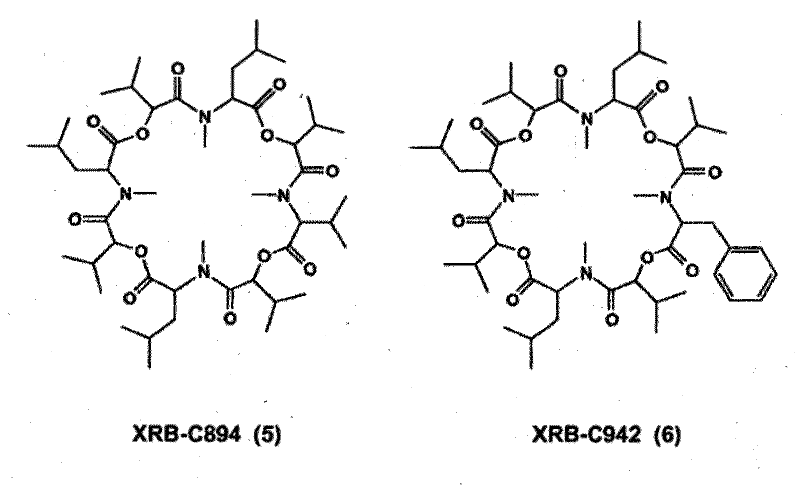

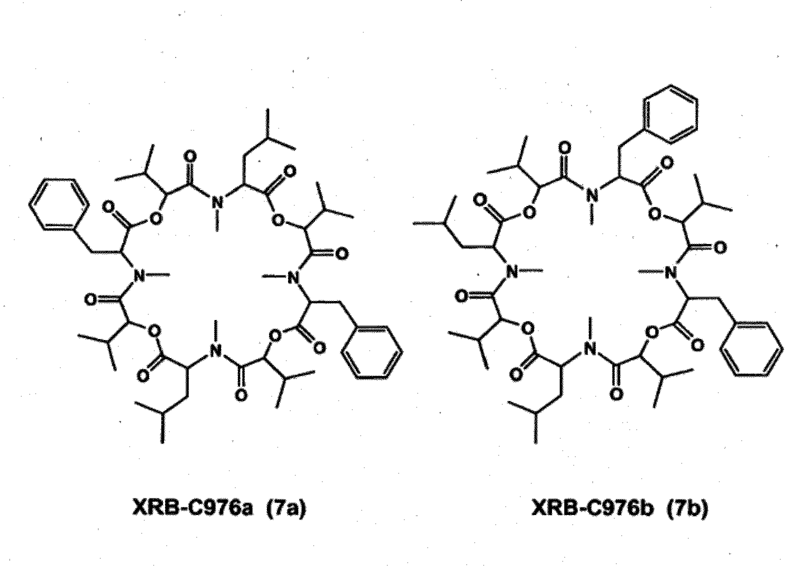

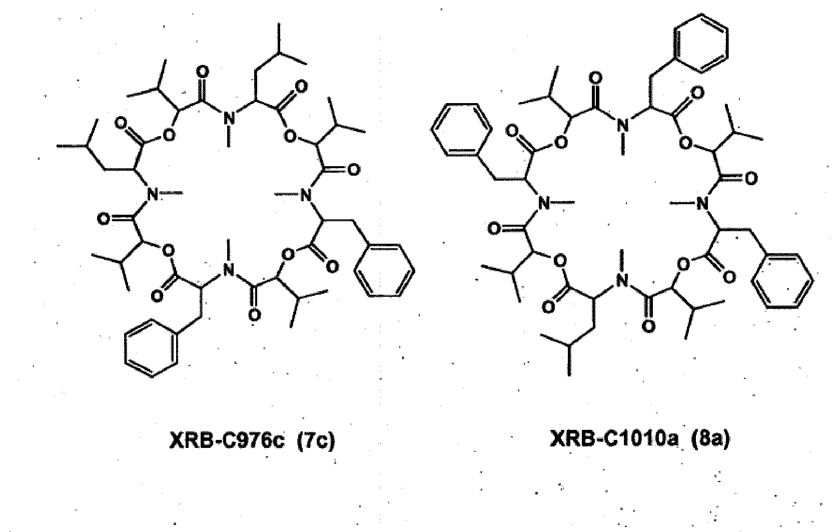

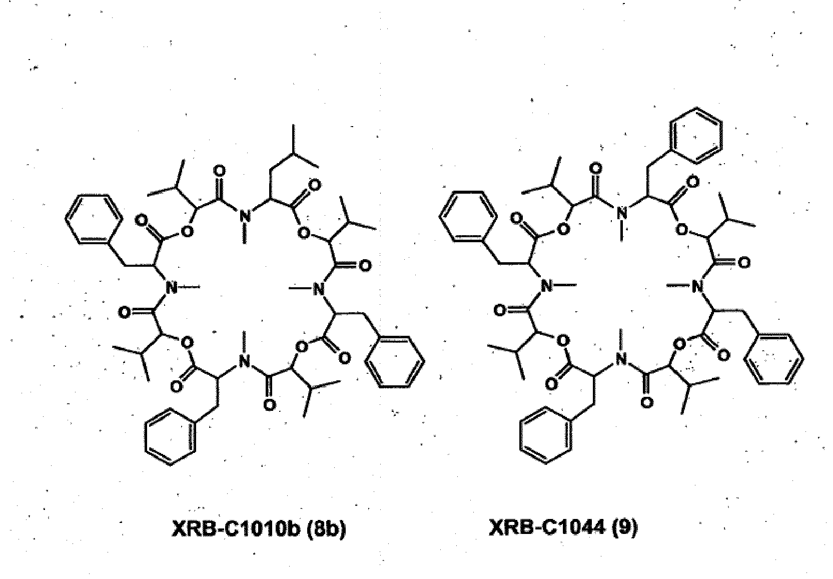

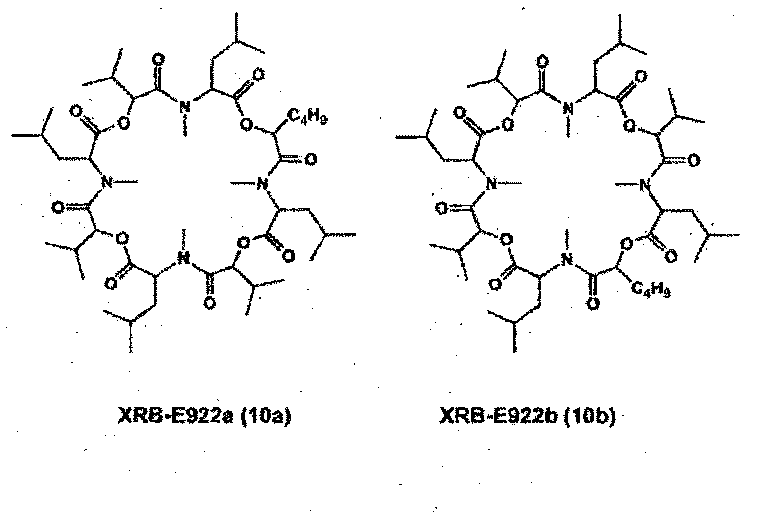

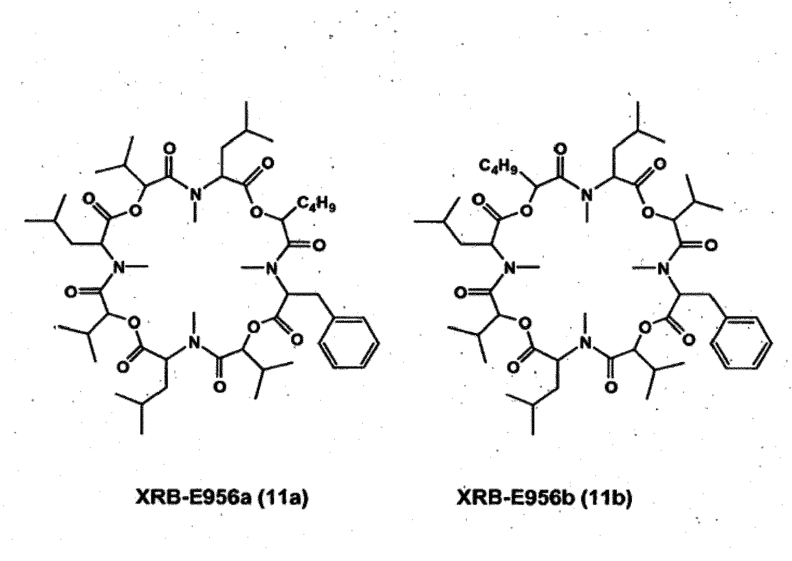

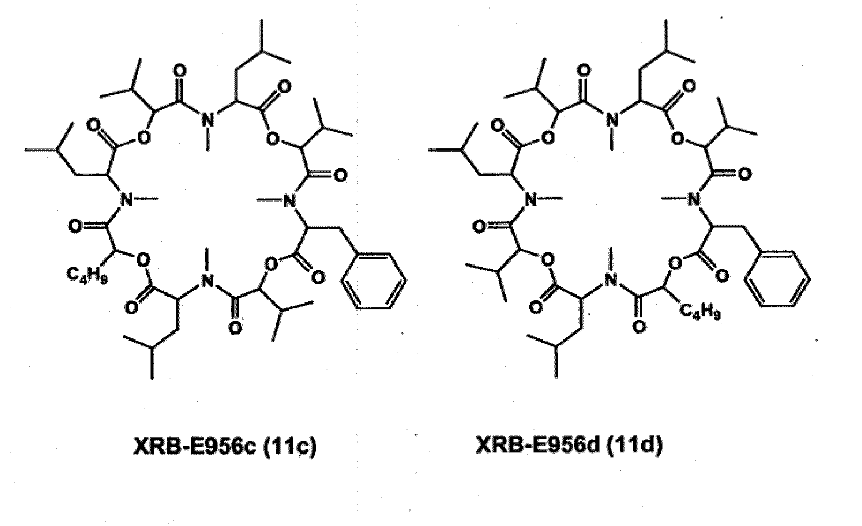

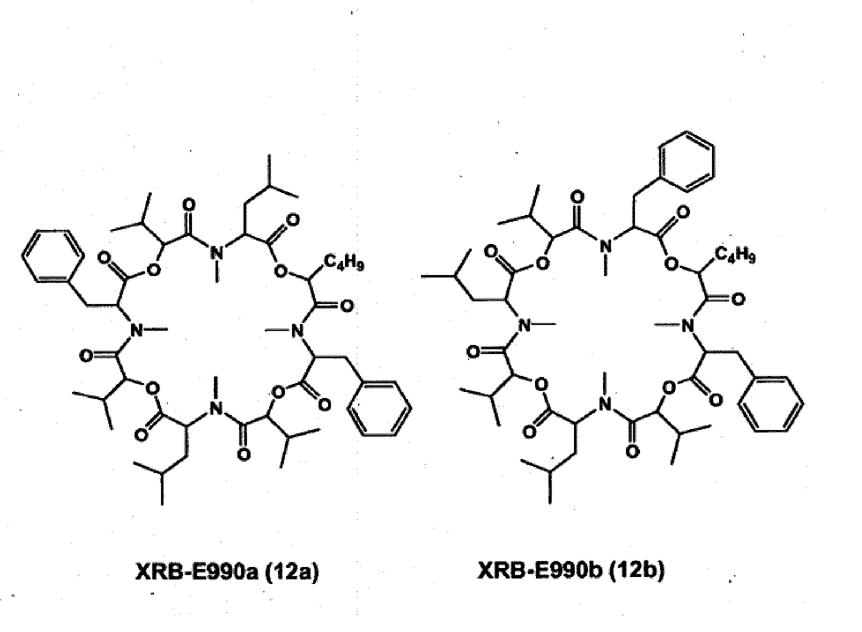

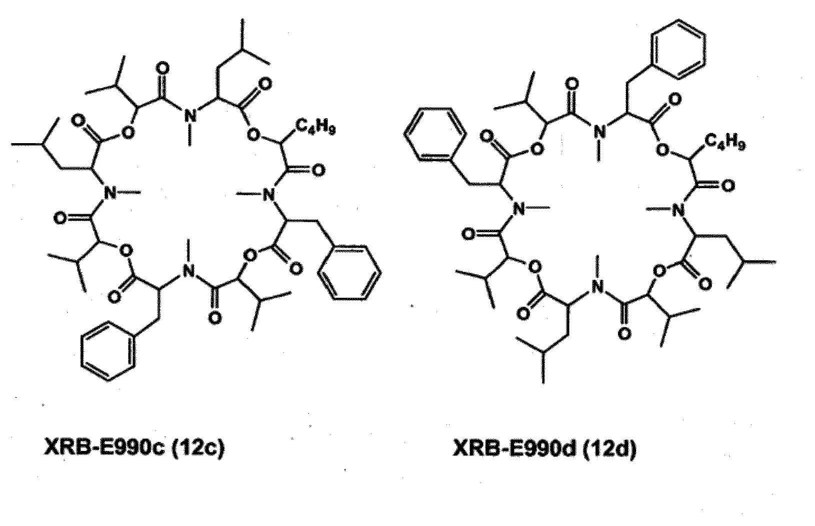

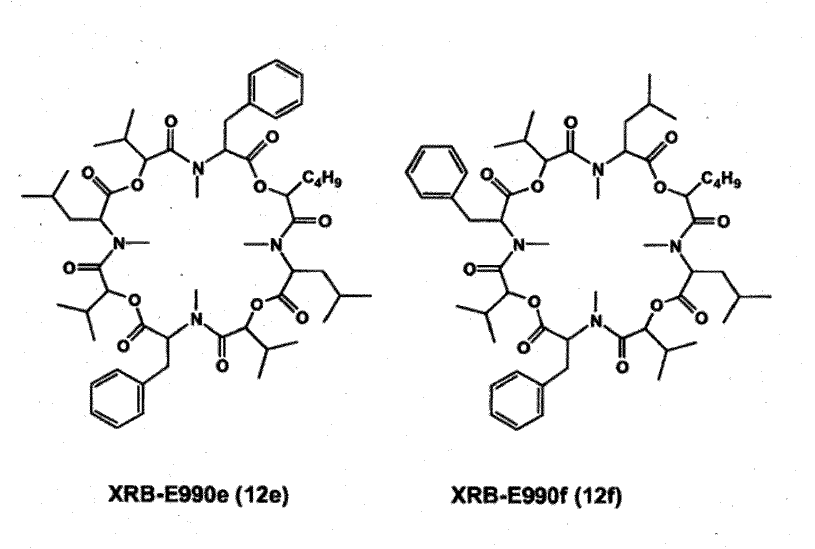

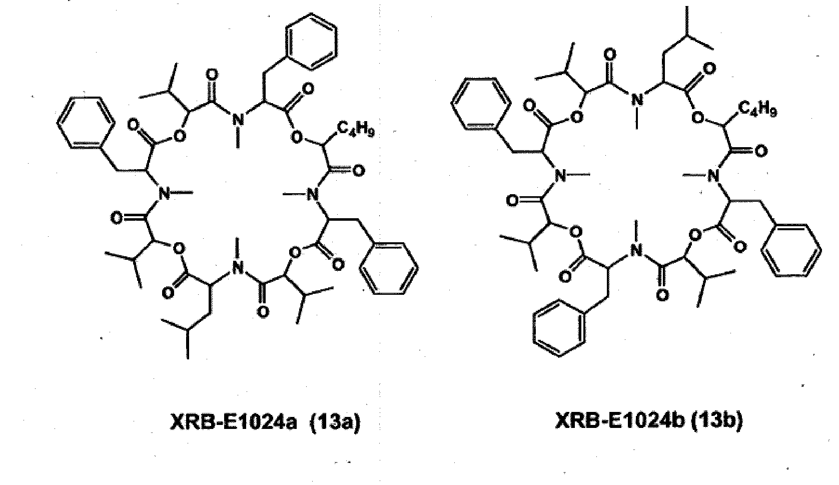

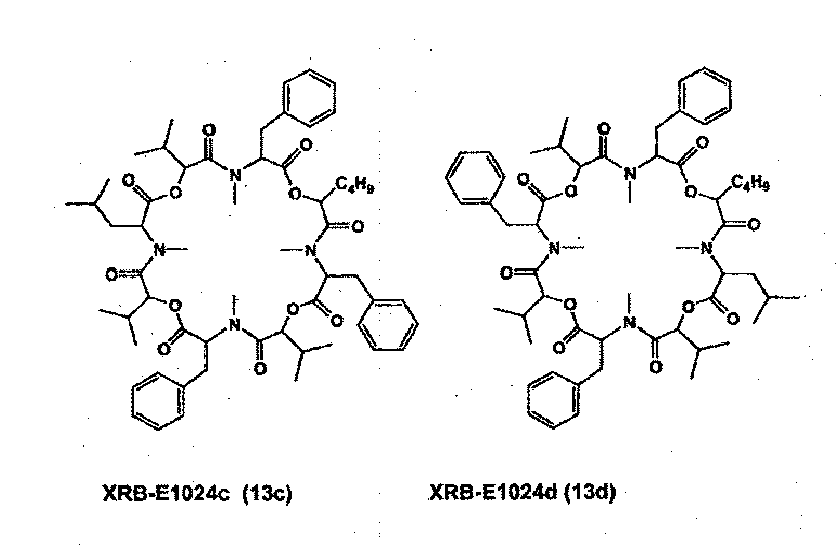

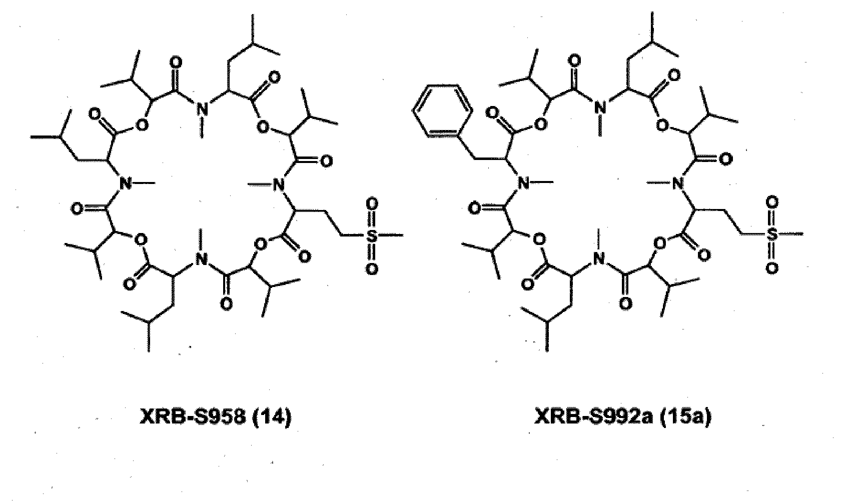

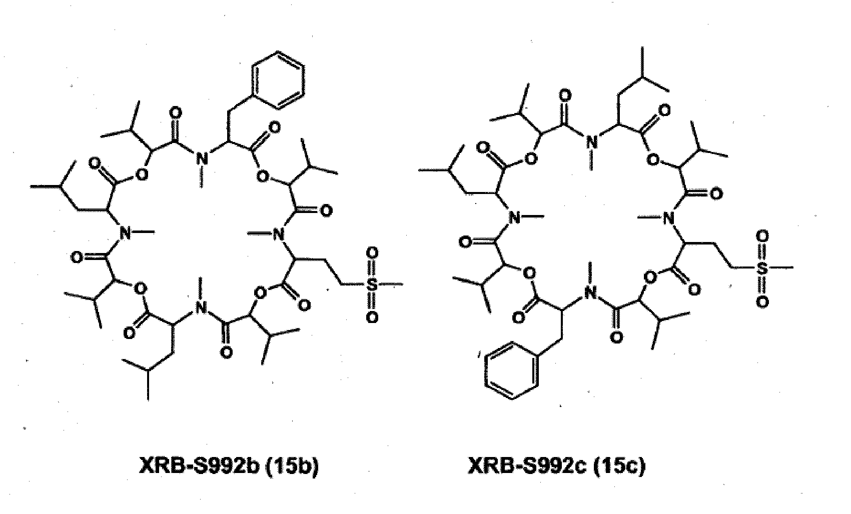

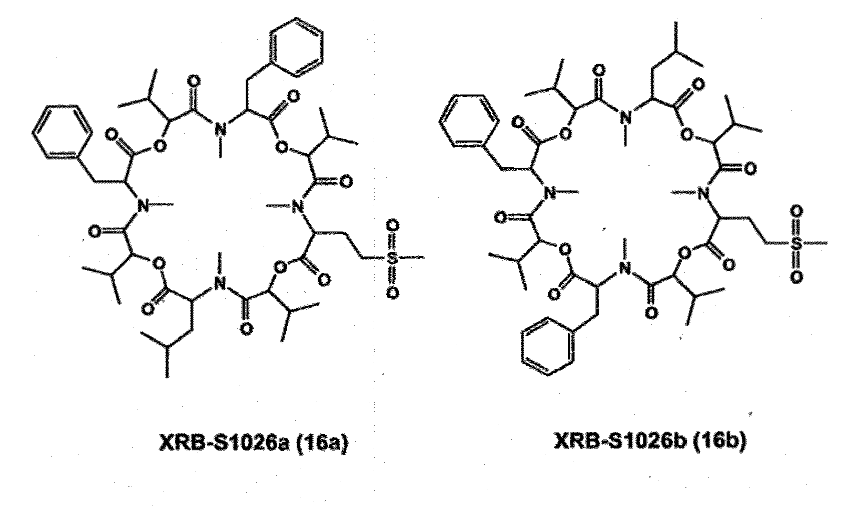

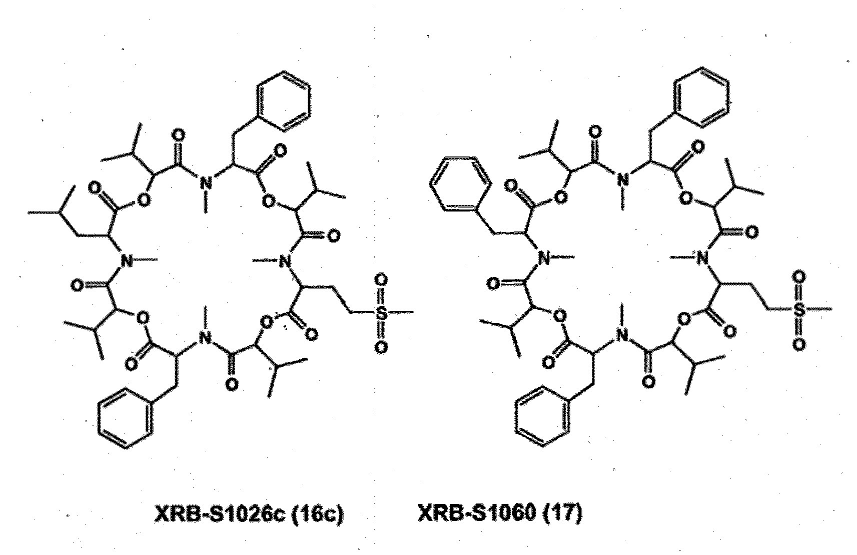

2. Cyclooctadepsipeptides selon la revendication 1 individuellement ou sous forme d'un mélange avec du bassianolide destinés à être utilisés comme anthelminthiques ou endoparasiticides.
